# EUROPEAN PATENT APPLICATION

(11) **EP 2 108 696 A1**
(43) Date of publication of application: **14.10.2009**
(21) Application number: 08007120.2
(22) Date of filing: 10.04.2008
(51) Int. Cl.: C12N 15/00, C12N 15/01, C12N 5/06

(54) **Cis-acting diversification activator and method for selective diversification of nucleic acids**

(71) Applicant: Helmholtz Zentrum München Deutsches Forschungszentrum für Gesundheit und Umwelt GmbH, 85764 Neuherberg (DE)
(72) Inventor: Caldwell, Randolph B., 80939 München (DE); Buerstedde, Jean-Marie, 80807 München (DE)
(74) Representative: UEXKÜLL & STOLBERG

(57) **Abstract**

The invention relates to identification of a cis-acting diversification activator *(DIVAC)* that is necessary and sufficient for the activation of diversification in transcription units linked thereto. The invention provides a method for diversification of a target nucleic acid comprising introducing a genetic construct comprising the diversification activator into a recipient cell, wherein the diversification activator is linked to the target nucleic acid.

## Description

The present invention relates to a method for selective diversification of a target nucleic acid sequence in a recombinant recipient cell by employing a regulatory sequence from the immunoglobulin locus that activates genetic diversification in linked transcription units.

### INTRODUCTION

Mutated and sequence optimized genes and gene products have extended application in medical, chemical and technical areas. Present strategies for diversification of genes and gene products rely mainly on the generation of a vast number of mutant sequences *in vitro*, artificial expression and subsequent selection for a desired property. These methods have the drawback that the mutated genes are difficult to express in the physiological context and their mutant repertoire is fixed in time when they are expressed. On the other hand, if genome wide mutagenesis is induced in living cells, this is accompanied by high toxicity due to accidentally lethal mutations and the mutation rates in individual genes are low, i.e., the mutagenesis lacks selectivity.

Accordingly, attempts were made to provide a genetic system that allows for locus specific nucleic acid diversification within living cells. In nature, directed and selective diversification is primarily employed to create diversity within the immunoglobulin genes. Thus, vertebrate B cells are able to diversify their rearranged immunoglobulin (Ig) genes by hypermutation, gene conversion and class switch recombination. All three phenomena require expression of Activation Induced cytidine Deaminase (AID, NC_006088) [Miramatsu et al., 2000; Arakawa et al., 2002; Harris et al., 2002], which most likely initiates *Ig* gene diversification by deaminating cytidines within the mutating and recombining sequences [Di Noia and Neuberger, 2002; Rada et al., 2004]. A further requirement for hypermutation and switch recombination is the transcription of the *Ig* genes and the switch regions respectively [Peters and Storb, 1996; Shinkura et al., 2003].

Sequence analysis of transcribed non-*Ig* genes from AID expressing B cells revealed either none or very few mutations compared to *Ig* genes [Shen et al., 1998]. A recent study of numerous expressed genes in B cells indicated that the number of mutations in wild-type mice is significantly higher than in AID knock-out mice [Liu et al., 2008]. However, the mutation rates for the *non-Ig* genes in AID expressing B cells were still orders of magnitude lower than for the *Ig* genes. To explain this difference between *Ig* and *non-Ig* genes it was suggested that *cis*-acting sequences in the *Ig* loci activate hypermutation possibly by recruiting AID. However, efforts to unambiguously define these sequences for the murine and human *Ig* loci were unsuccessful [Odegard and Schatz, 2006]. Studies using chimeric reporter genes in transgenic mice indicated that certain *Ig* enhancers and their surrounding sequences conferred hypermutation activity [Betz et al., 1994; Klotz and Storb, 1996; Klix et al., 1998]. However, deletion of *Igk* enhancers in knock-out mice did not prevent hypermutation of the *Igk* gene (CAA36032) [van der Stoep et al., 1998; Inlay, 2006]. At least one murine B cell line [Wang et al., 2004] and AID expressing fibroblasts [Yoshikawa et al., 2002] mutated transcribed transgenes in the absence of nearby *Ig* locus sequences, further confounding the issue of whether *cis*-acting regulatory sequences are needed for hypermutation.

Previously the inventors demonstrated that the chicken B cell line DT40 diversifies its rearranged *Ig light chain* (*IgL*) gene by gene conversion in the presence of nearby *pseudo V* (ψ*V*) genes [Arakawa et al., 2002] or by hypermutation, if the ψ*V* genes are deleted [Arakawa et al., 2004; WO 2005/080552]. Both activities strictly depend on the expression of AID. Subsequently, the inventors showed that a Green *Fluorescent Protein* (*GFP*, AAB08058) transgene is rapidly diversified by mutations when inserted into the rearranged *IgL* locus [Arakawa et al., 2008], consistent with the initial finding that the deletion of homologous gene conversion donors leads to hypermutation of the IgL gene [Arakawa et al., 2004]. At the same time, no mutations were found in the highly transcribed *Elongation Factor 1* alpha gene (NP_989488) [Arakawa et al., 2004], indicating that hypermutation occurs only within the IgL locus. Also the *VpreB3* (NC_006102) or the *Carbonic Anhydrase* (XP_415218) gene, upstream and downstream neighbors of the *IgL* locus, respectively, showed no sequence heterogeneity in DT40 [Gopal and Fugmann, 2008].

It was also observed that the inactivation of the E2A transcription factor gene reduces the rate of *IgL* chain mutations without affecting the levels of surface Ig or AID expression, suggesting that E2A-encoded proteins enhance Ig hypermutation by recruitment of AID to the *Ig* loci [Schoetz et al., 2006]. In addition, a deletion of the rearranged *IgL* locus downstream of the C region stopped *IgL* gene conversion in ψ*V* positive DT40 [Kothapalli et al., 2008], indicating that parts of the IgL locus are necessary for gene conversion.

However, none of the known studies either in human, murine or DT40 cells revealed a *cis*-acting regulatory sequence that is both necessary and sufficient for the Ig locus specificity of AID-dependent genetic diversification. Such a sequence would allow generating diversity of nucleic acid target sequences outside the Ig loci in AID expressing cells.

Therefore, there exists a need for a regulatory sequence that activates diversification in the nucleic acids linked thereto and a method to achieve specific diversification of a target nucleic acid. The present invention satisfies the need and provides further advantages as well.

### SUMMARY OF THE INVENTION

The invention makes available a regulatory nucleic acid molecule, a *cis*-acting diversification activator (*DIVAC*), that has the function of activating diversification in transcription units linked thereto. A diversification activator of the invention is derived from the immunoglobulin (Ig) locus of a chicken B cell and comprises a nucleic acid having the sequence SEQ ID NO:1, a fragment of said nucleic acid or a nucleic acid homologous to SEQ ID NO:1 or a fragment thereof, whereby said fragment and said homologue retain the *DIVAC* function.

Accordingly, the invention provides a method for diversification of a target nucleic acid comprising introducing a genetic construct comprising a diversification activator of the invention into a recipient cell to produce a recombinant recipient cell, wherein said diversification activator is linked to the target nucleic acid.

A target nucleic acid can be supplied to the recipient cell from outside as a transgene, or be part of the recipient cell genome. In each case, it is secured that the target nucleic acid is linked to *DIVAC*, i.e., DIVAC activates and directs the diversification of the target nucleic acid.

The invention further provides a method for producing a target nucleic acid having a desired activity, comprising the steps of (a) transfecting or infecting a recipient cell one or more times with one or more genetic construct(s) comprising a diversification activator and, optionally, the target nucleic acid to obtain a recombinant recipient cell, (b) identifying a recombinant recipient cell, wherein said diversification activator is linked to said target nucleic acid, (c) propagating and expanding the cell from (b) under conditions appropriate for the expression and diversification of the target nucleic acid, and (d) selecting within the population of cells from (c) individual cells or cell populations containing a mutated target nucleic acid having a desired activity.

The invention further provides a nucleic acid construct comprising a diversification activator of the invention and a recombinant recipient cell comprising a diversification activator of the invention integrated into the cell chromosome at a position different from the immunoglobulin (Ig) locus.

### DESCRIPTION OF THE FIGURES

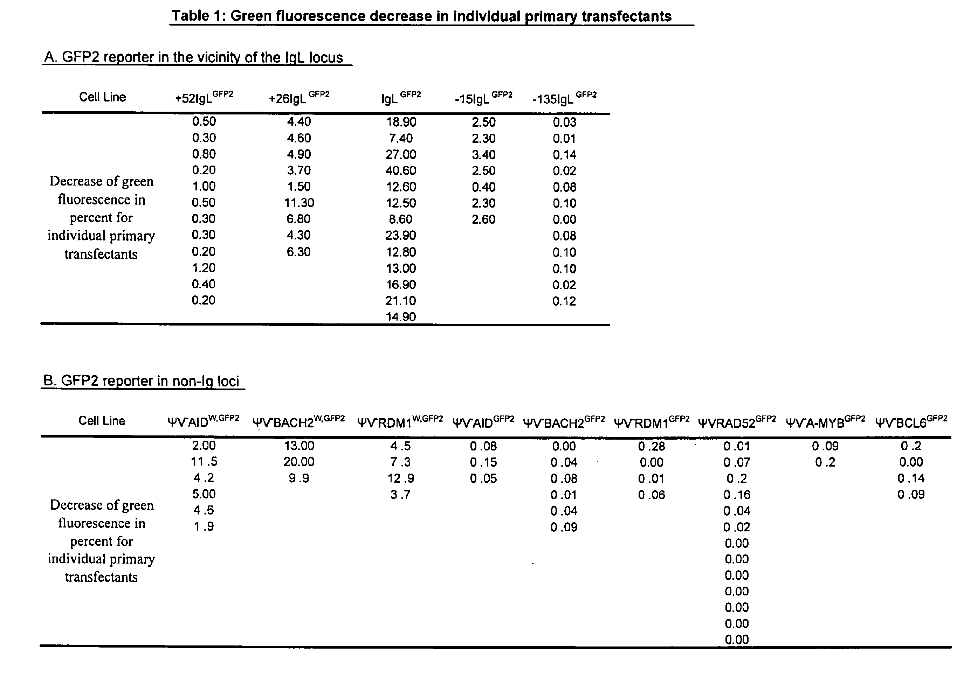

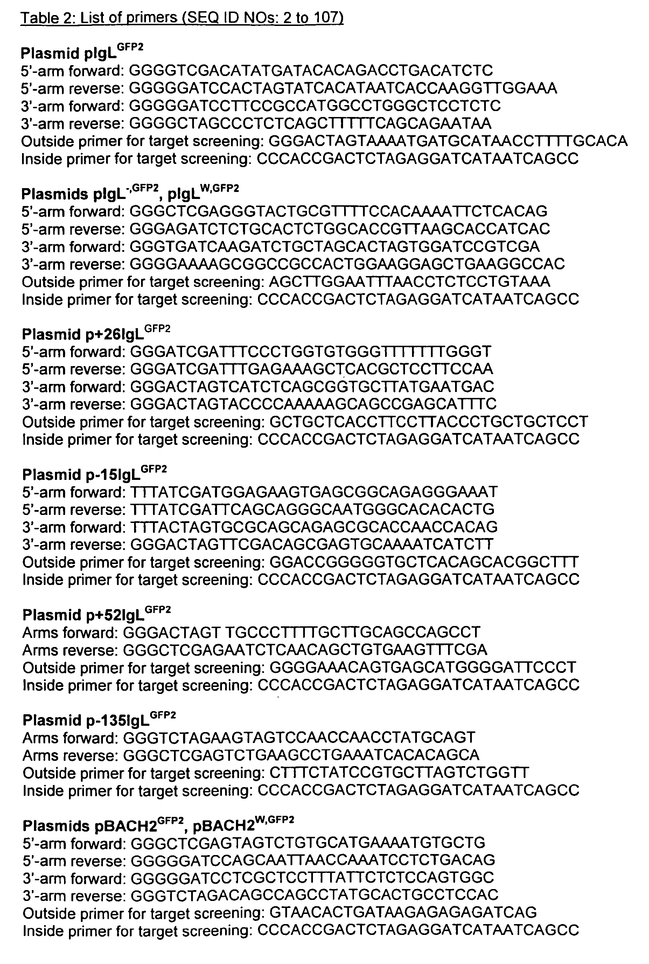

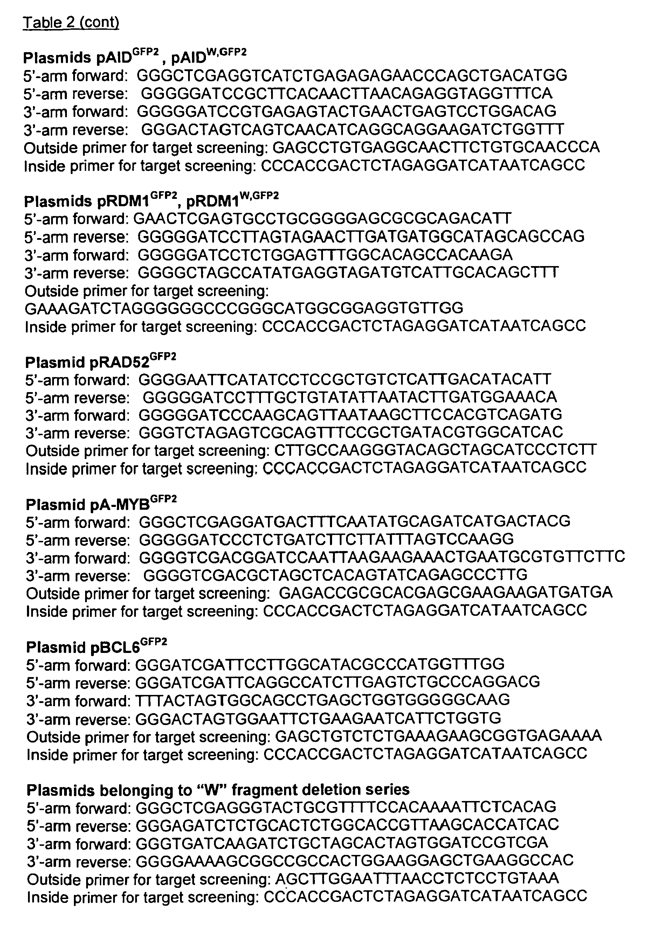

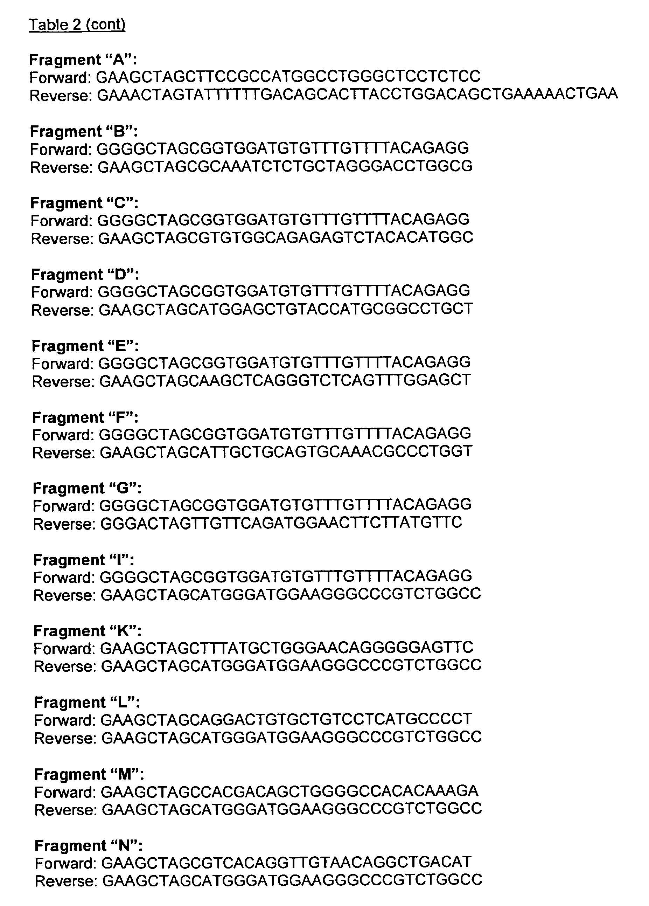

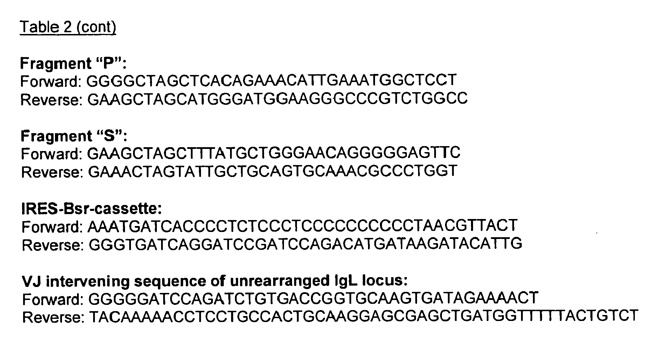

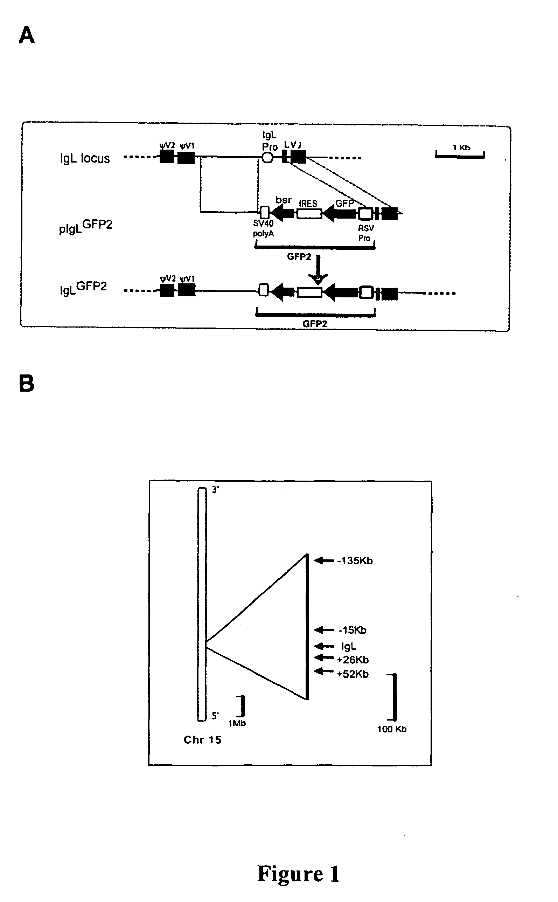

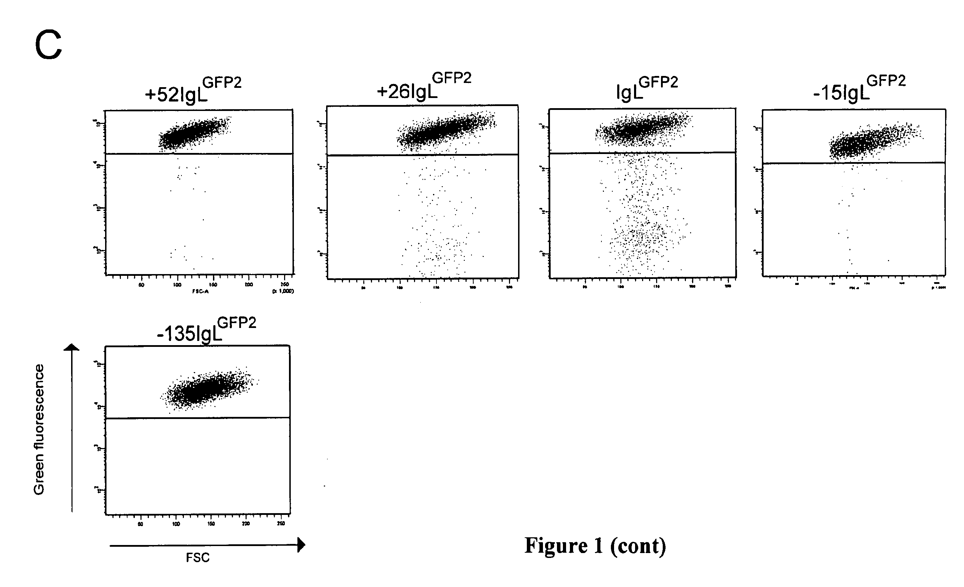

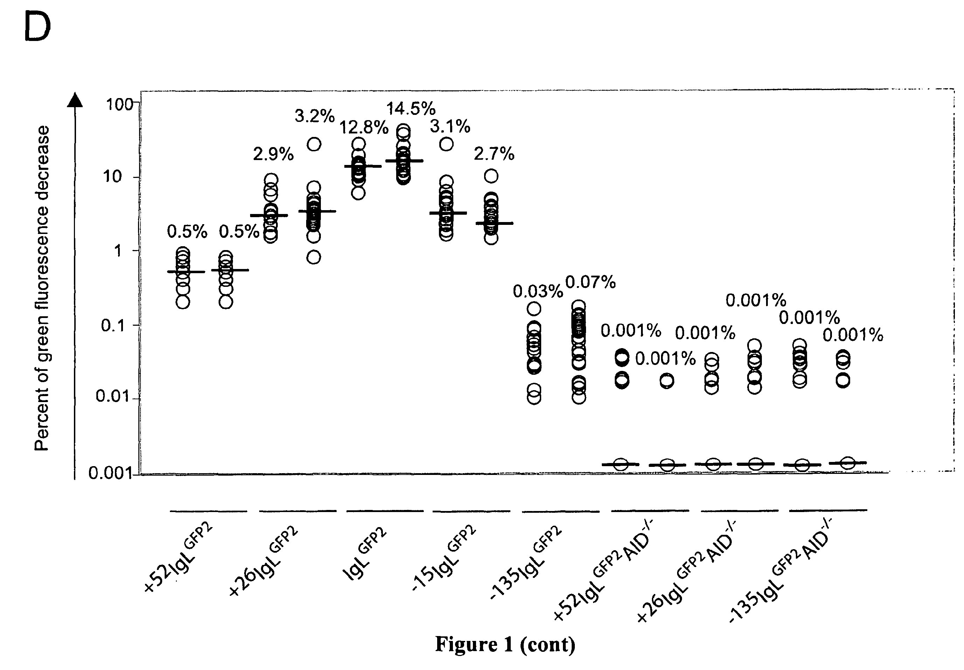

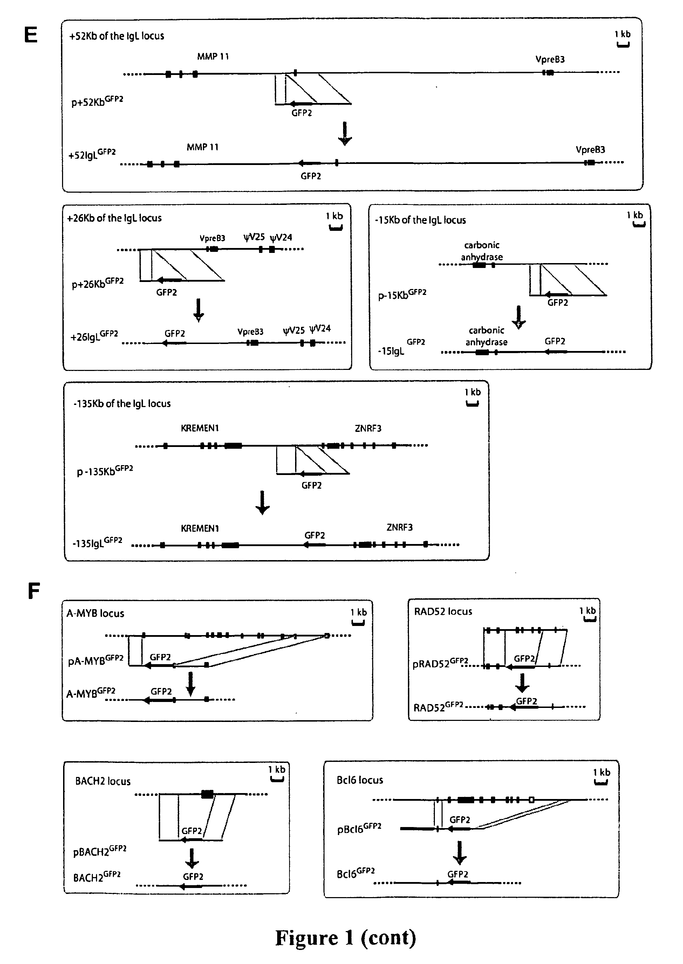

Figure 1. Hypermutation of the *GFP2* reporter at various distances from the rearranged *IgL* locus. (A) A physical map of the rearranged *IgL* locus, a targeting construct including the *GFP2* reporter and the *IgL* locus after targeted insertion of the *GFP2* reporter. (B) Locations of *GFP2* insertion relative to the *IgL* locus on chromosome 15. The reference point is the insertion site of *GFP2* in the IgL^{GFP2} transfectant. (C) FACS analysis of primary transfectants having integrated the transfected construct targeted. (D) Fluctuation analysis of subclones. Each dot represents the analysis of and the median of all subclones from the same primary transfectant is indicated by a bar. (E) Targeted integration of *GFP2* containing constructs at various distances from the *IgL* locus into chromosome 15 [22]. (F) Targeted integration of *GFP2* containing constructs at the A-MYB, RAD52, BACH2 and Bc16 loci.

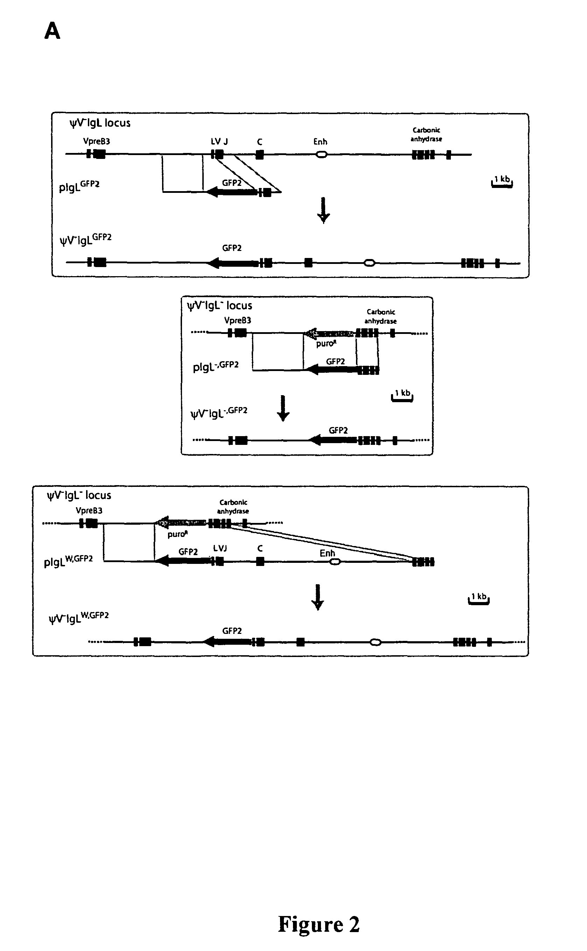

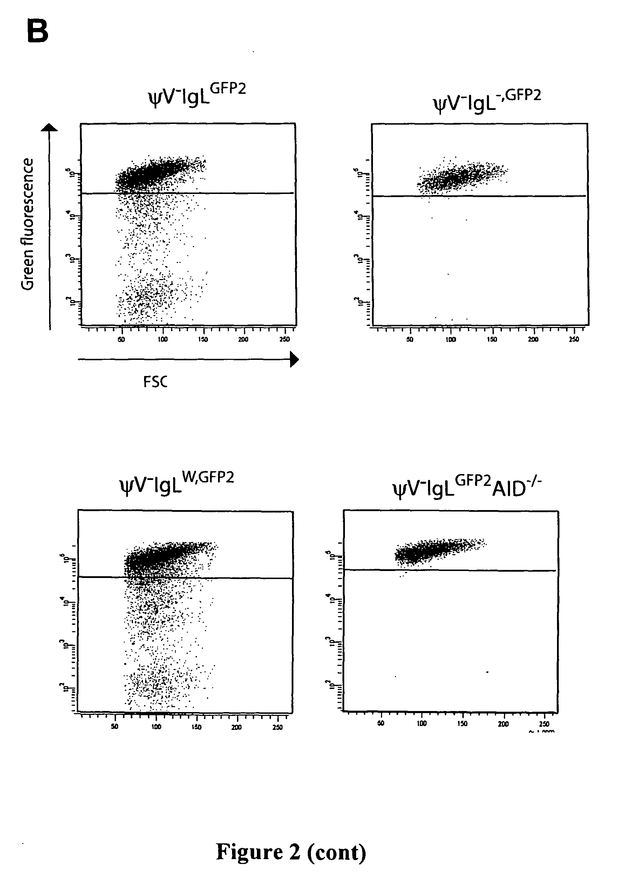

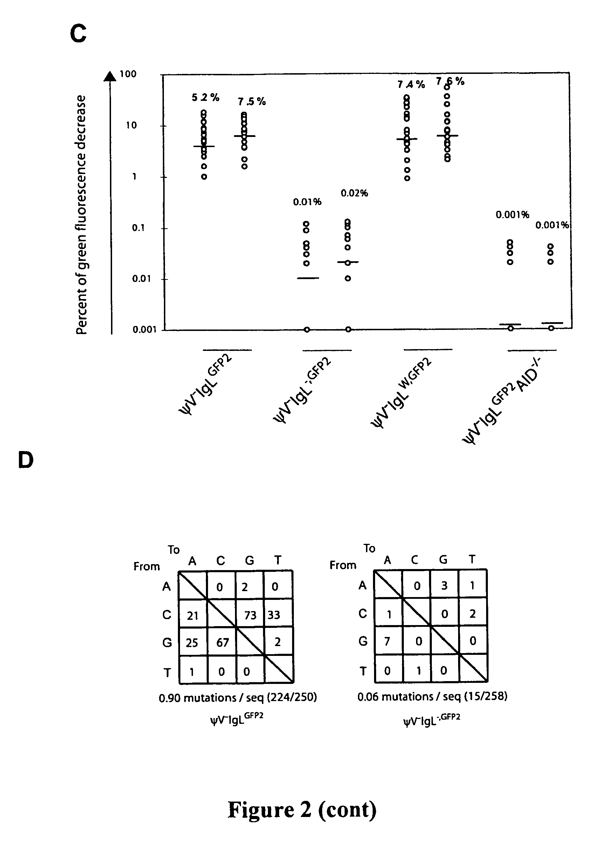

Figure 2. *GFP2* hypermutation after deletions and reconstitution of the rearranged *IgL* locus. (A) Design of deletions and insertions in the rearranged *IgL* locus using *GFP2* targeting constructs. (Upper part) Physical maps of the rearranged *IgL* locus after the deletion of the ψ*V* locus, a targeting construct including the *GFP2* reporter and the locus after targeted integration. (Middle part) Physical maps of the deleted rearranged *IgL* locus, a targeting construct including the *GFP2* reporter and the *GFP2* insertion site after targeted integration. (Lower part) Physical maps of the deleted rearranged *IgL* locus, a targeting construct including the *GFP2* reporter together with the '*W*' fragment and the *GFP2* insertion site in the reconstituted *IgL* locus. (B) FACS analysis of primary transfectants. (C) Fluctuation analysis of subclones. (D) The frequencies of particular nucleotide substitutions within the *GFP* open reading frame of *GFP2.*

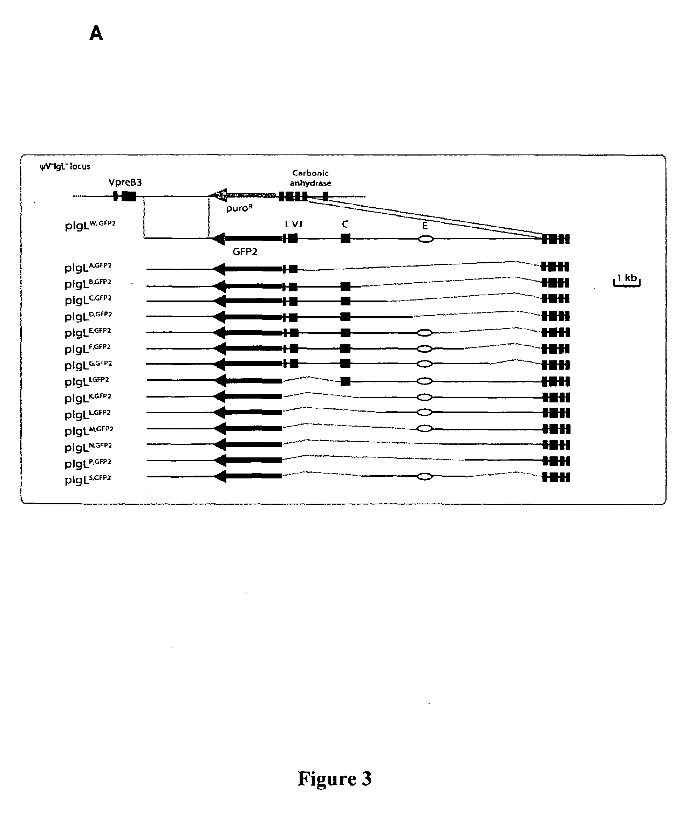

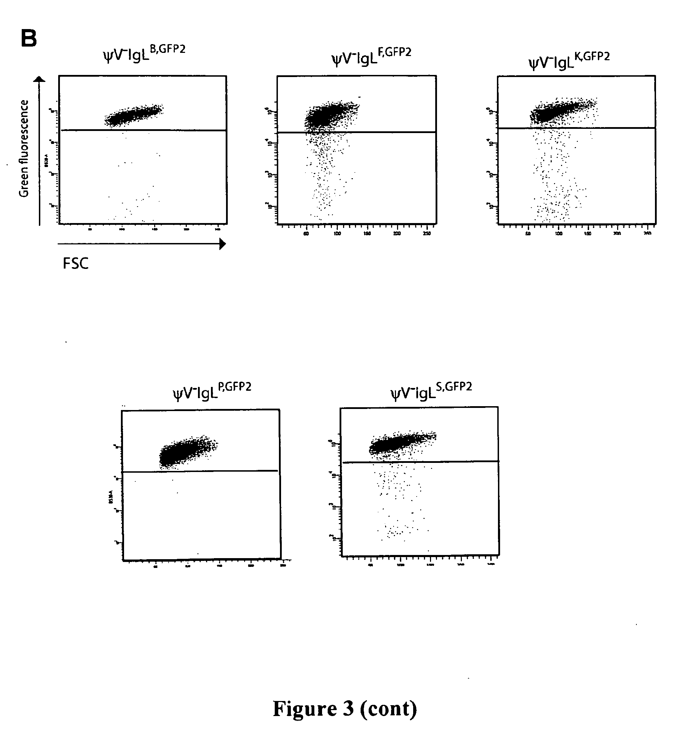

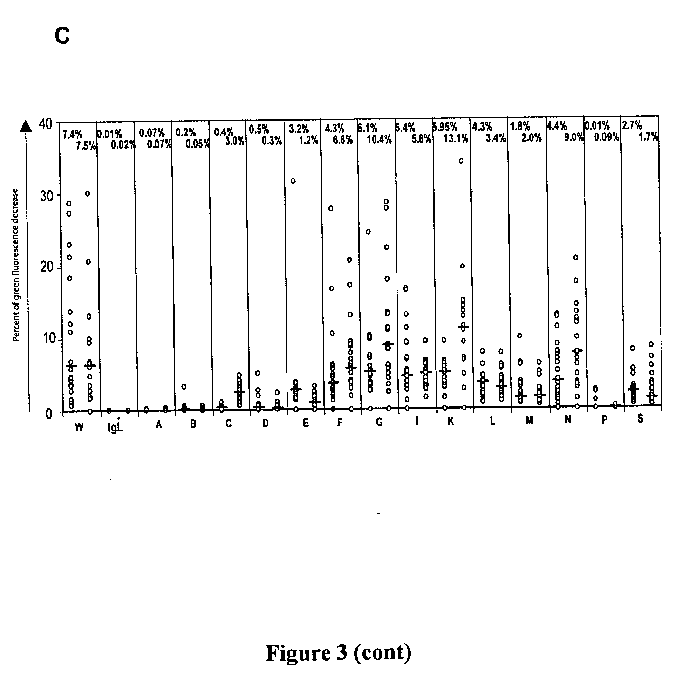

Figure 3. Analysis of the '*W*' fragment deletion series. (A) A physical map of the deleted *IgL* locus and the aligned targeting constructs leading to the insertion of the *GFP2* reporter together with parts of the '*W*' fragment. (B) FACS analysis of representative primary transfectants. (C) Fluctuation analysis of subclones. Only the letter of the reconstituted fragment and not the full name of the transfectants is indicated.

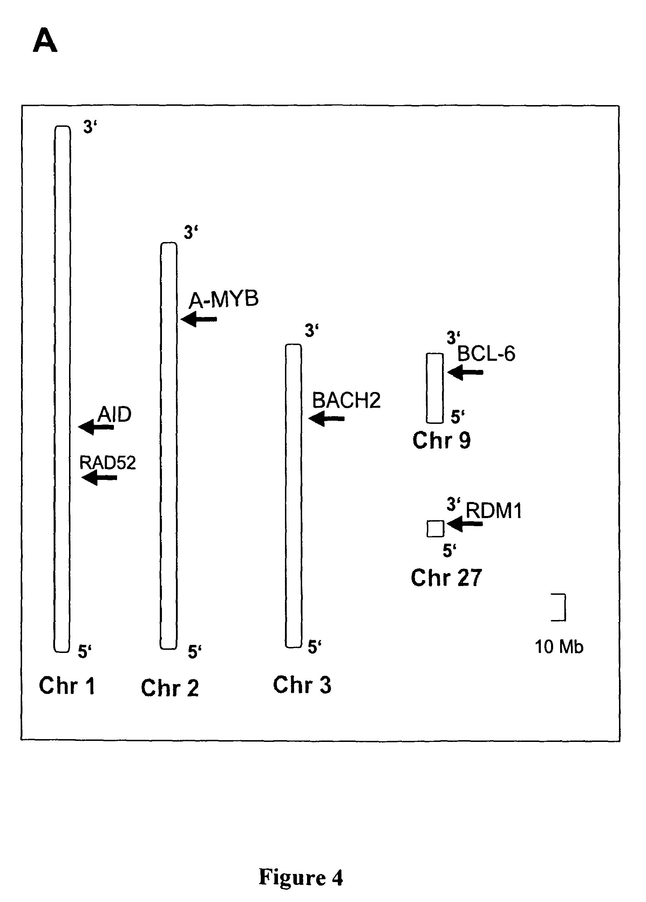

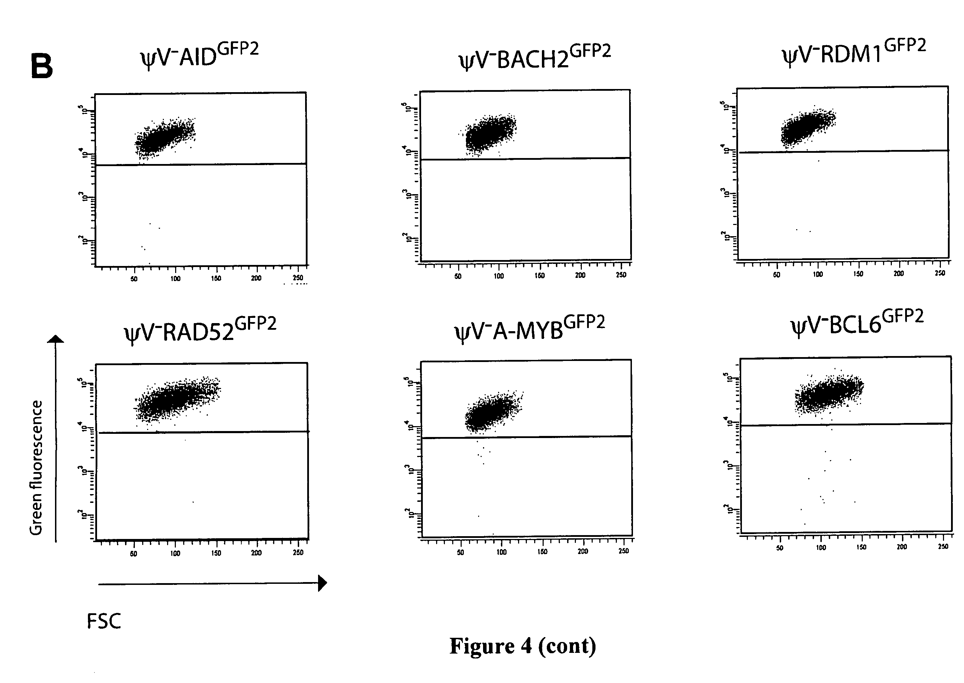

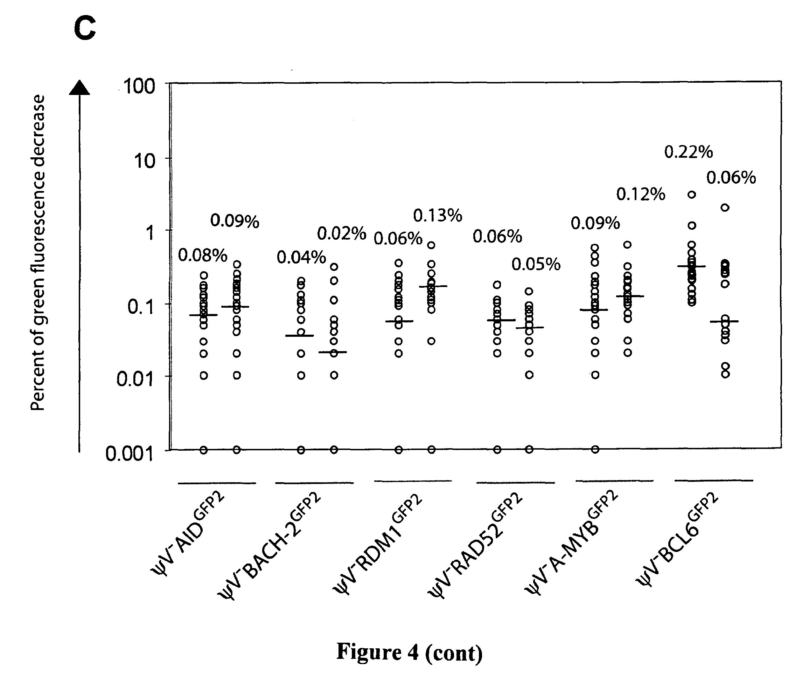

Figure 4. Insertions of the *GFP2* reporter into *non-Ig* loci. (A) Chromosomal locations of the *GFP2* insertions. (B) FACS analysis of primary transfectants. (C) Fluctuation analysis of subclones.

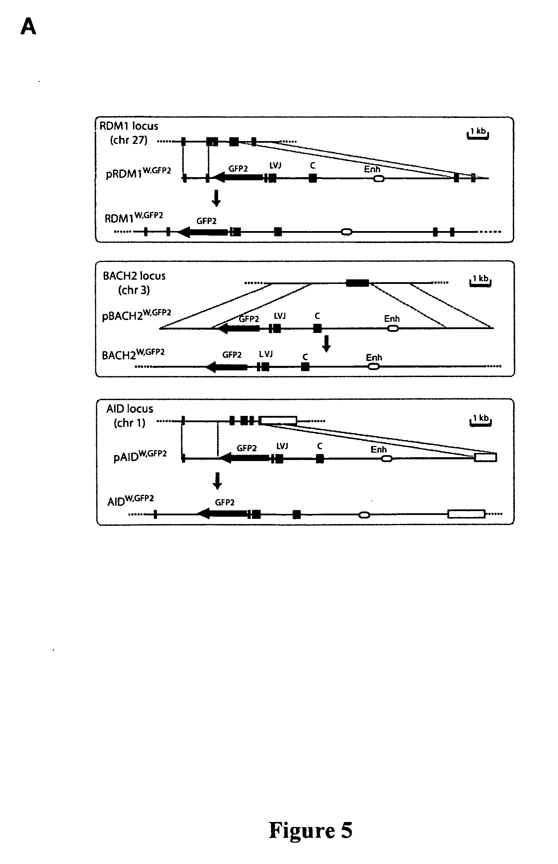

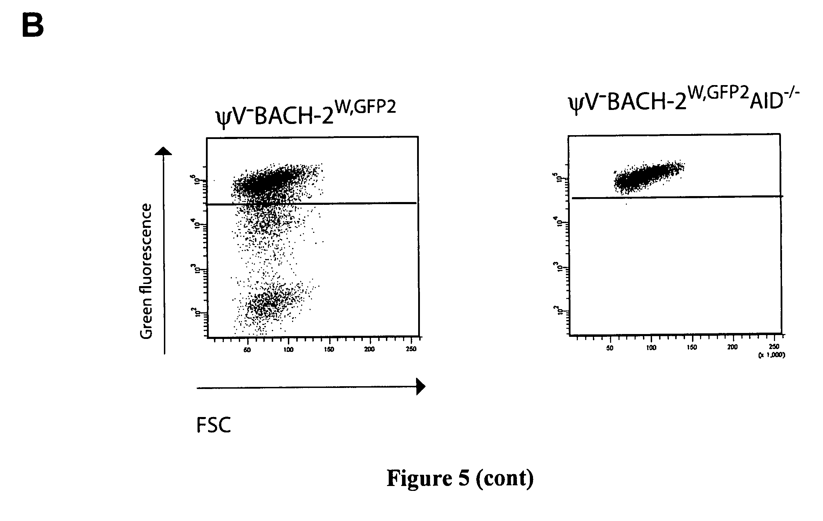

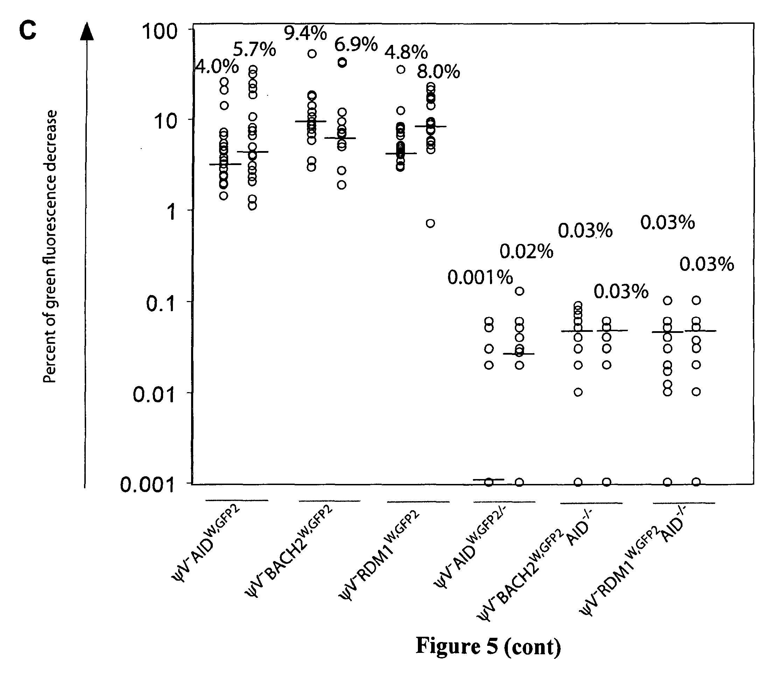

Figure 5. Hypermutation of the *GFP2* reporter at *non-Ig* loci in the presence of the '*W*' fragment. (A) Physical maps of the *non-Ig* loci, the targeting constructs including the *GFP2* reporter together with the '*W*' fragment and the loci after targeted insertion of the *GFP2* reporter. (B) FACS analysis of primary *AID* positive and *AID* negative transfectants. (C) Fluctuation analysis of subclones.

### DETAILED DESCRIPTION OF THE INVENTION

The present invention makes available an improved method for genetic diversification of a target nucleic acid within a living cell. The method is based on the discovery of a regulatory nucleic acid molecule, a *cis*-acting diversification activator (*DIVAC*), that is essential and sufficient for the activation of genetic diversification of a nucleic acid that is linked thereto.

For the purpose of the invention the following definitions apply. "Genetic diversification" is alteration of individual nucleotides or stretches of nucleotides in a target nucleic acid. Genetic diversification according to the invention preferably occurs by hypermutation. In the event that in the method of the invention homologous donors for the target nucleic acid are provided, genetic diversification may occur by gene conversion or a combination of hypermutation and gene conversion.

"Hypermutation" diversifies a target nucleic acid primarily by single nucleotide substitutions. Preferably, hypermutation refers to a rate of mutation of between 10⁻⁵ and 10⁻³ bp⁻¹ generation⁻¹ which at least 100 fold higher than the background mutation rate in non-hypermutating genes within the same cell.

"Gene conversion" is a phenomenon in which sequence information is transferred in unidirectional manner from a homologous gene conversion donor to a gene conversion target sequence. Gene conversion may be the result of a DNA polymerase switching templates and copying from a homologous sequence, or the result of mismatch repair (nucleotides being removed from one strand and replaced by repair synthesis using the other strand) after the formation of a heteroduplex. An example of natural gene conversion is the diversification of the rearranged VJ IgL segments by nearby pseudo-V gene conversion donors in certain species.

In many vertebrates, hypermutation and gene conversion generate diversity within the immunoglobulin V(D)J segment of B cells. Hypermutation takes place in the germinal centers of such species as mouse and human following antigen stimulation. Gene conversion takes place in primary lymphoid organs like the Bursa of Fabricius or the gut-associated lymphoid tissue in such species as chicken, cow, rabbit, sheep and pig independent of antigen stimulation. "Diversification activator" is a *cis*-acting regulatory sequence that activates the genetic diversification of neighboring transcription units which behave as target nucleic acids.

"Target nucleic acid" is a nucleic acid molecule subjected to genetic diversification. The target nucleic acid is preferably a transgene and may comprise one or more transcription units encoding gene products. The target nucleic acid may also be a non-coding transcription unit or an endogenous gene whose diversification is activated by the nearby insertion of a diversification activator. The target nucleic acid altered as a result of *DIVAC*-related diversification is also called mutant nucleic acid.

"Transgene" is an exogenous nucleic acid molecule that is inserted into the recipient cell, such as by transfection, transduction, or infection. For example, a transgene may comprise a heterologous transcription unit which may be inserted into the genome randomly or at a defined location by targeted integration. For the purpose of the invention the transgene is preferably located next to a diversification activator thereby becoming a target nucleic acid.

"Targeted integration" is integration of a transfected nucleic acid construct comprising a nucleic acid sequence homologous to an endogenous nucleic acid sequence by homologous recombination into the endogenous locus. Targeted integration allows to directly insert any nucleic acid into a defined chromosomal position.

"Recombinant recipient cell" refers to a cell that is engineered for genetic diversification of a target nucleic acid. Preferably, the recipient cell expresses AID and is the recipient of the transgene linked to the diversification activator.

"Selection" refers to the determination of the presence of sequence alterations in the target nucleic acid that result in a desired activity of the gene product encoded by the target nucleic acid or in a desired activity of the regulatory function of the target nucleic acid.

In one aspect, there is provided a method for diversification of a target nucleic acid comprising introducing a genetic construct comprising a diversification activator into a recipient cell to produce a recombinant recipient cell,
wherein said diversification activator is linked to the target nucleic acid,
wherein said diversification activator is selected from the group consisting of a nucleic acid having the sequence SEQ ID NO:1, a fragment of said nucleic acid and a nucleic acid homologous to said nucleic acid and said fragment, and
wherein said diversification activator has the function of activating genetic diversification in a nucleic acid linked thereto.

In one embodiment, the diversification takes place by the process of somatic hypermutation. A preferred rate of somatic hypermutation is between 10⁻⁵ and 10⁻³ bp⁻¹ generation⁻¹.

In another embodiment, the target nucleic acid is in addition linked to at least one nucleic acid capable of serving as a gene conversion donor for the target nucleic acid, and the diversification takes place by a process involving both somatic hypermutation and gene conversion.

A diversification activator (*DIVAC*) of the invention is derived from the immunoglobulin (*Ig*) locus of a chicken B cell. In one embodiment, *DIVAC* is a 9,8 kb fragment of the rearranged *IgL* locus extending from the *IgL* transcription start site to the 3' end of the carbonic anhydrase gene. The 9,8 kb fragment maps to *Gallus gallus* chromosome chr15:8165070-8176699. The 9,8 kb fragment isolated from the chicken B cell line DT40 has the nucleotide sequence SEQ ID NO:1. *DIVAC* can also be a functional homologue of said 9,8 kb *IgL* fragment from the chicken *Ig heavy chain* (*IgH*) locus.

According to the invention, the *DIVAC* function of activating diversification of a linked target nucleic acid is defined by an at least 5-fold, 10-fold, 20-fold, 50-fold or 100-fold increase of the diversification rate of the target nucleic acid in the presence of *DIVAC* compared to that in the absence of *DIVAC*.

In another embodiment, *DIVAC* of the invention is a fragment of the 9,8 kb fragment that retains the DIVAC function of activating diversification of a nucleic acid linked thereto. The *DIVAC* fragment may be as short as 20 bp and as long as 5000 bp. For example, the length of *DIVAC* may be at least 20 nucleic acids, at least 100 nucleic acids, at least 200 nucleic acids, at least 300 nucleic acids, at least 400 nucleic acids, at least 500 nucleic acids, at least 600 nucleic acids, at least 1000 nucleic acids, at least 2000 nucleic acids, at least 3000 nucleic acids, at least 4000 nucleic acids, or at least 5000 nucleic acids.

Specific *DIVAC* fragments may be defined on the basis of the oligonucleotides listed in Table 2 that may be used for PCR amplification of individual fragments. DIVAC fragments may also be defined on the basis of the nucleotide positions within the 9,8 kb fragment (SEQ ID NO:1).

Thus, *DIVAC* may have the nucleotide sequence between positions 1-7099, 1-7799, 2184-9784, 3098-9784, 4102-9784, 5114-9784, 6107-9784, or 3098-7099 of SEQ ID NO:1. These sequences correspond to the fragments designated F, G, I, K, L, M, N and S, respectively, on Figure 3A. However, DIVAC may be as short as a stretch of 20 to 50 nucleotides from SEQ ID NO:1 or the fragments described above.

In a further embodiment, *DIVAC* is a homologue of the 9,8 kb fragment or fragments thereof as defined above, whereby the homologue retains the *DIVAC* function of activating diversification of a nucleic acid linked thereto. For example, *DIVAC* of the invention shares at least 50%, at least 60%, at least 70%, at least 80%, at least 90%, or at least 95% sequence identity with SEQ ID NO:1 of a fragment thereof.

To increase the rate of diversification, *DIVACs* of the invention may be combined. For example, more than one copy of *DIVAC* may be provided to a recipient call in form of tandem repeats.

A target nucleic acid of the invention is a arbitrary nucleic acid whose diversification is desired. In the method of the invention, the target nucleic acid is preferably transcribed. In one embodiment, the target nucleic acid encodes a protein. For example, a target nucleic acid encodes an immunoglobulin chain, a selection marker, a DNA-binding protein, an enzyme, a receptor protein, or parts thereof. It is preferred that the target nucleic acid is a human immunoglobulin gene. The method of the invention would thus allow to produce an immunoglobulin gene encoding an immunoglobulin with a specific binding activity such as high specificity and/or high affinity to an antigen. It is also preferred that the target nucleic acid is a fluorescent protein such as Green Fluorescent Protein (GFP) or Yellow Fluorescent Protein (YFP). In this embodiment, the method of the invention would allow to produce an fluorescent protein with a new and different absorption/emission wave length.

In another embodiment, the target nucleic acid possesses a regulatory activity. For example, the target nucleic acid may be a transcription regulatory element such as promoter or enhancer, or encode an interfering RNA molecule for use in specific gene suppression by RNA interference. In this embodiment, a reporter, which is influenced by the target nucleic acid, is required for the identification of a recombinant recipient cell bearing the target nucleic acid of interest.

In one embodiment, the target nucleic acid is a exogenous nucleic acid. An exogenous nucleic acid may be provided to the recipient cell together with *DIVAC* on the same genetic construct, or, alternatively, on a different genetic construct or constructs. In this embodiment, the genetic construct(s) preferably comprise(s) a selectable marker gene that allows for selection of cells comprising the transgene and/or DIVAC. The selectable marker gene may subsequently be removed by recombination, for example using a cre-recombinase system, or inactivated by other means. It is preferred that the target nucleic acid and *DIVAC* stably integrate into the cell chromosome. Alternatively, the target nucleic acid and *DIVAC* may remain in the recipient cell extrachromosomally, for example, on an autonomously replicating viral vector such as an EBV-derived vector.

In another embodiment, the target nucleic acid is a *priori* a part of the cell chromosome, i.e., an endogenous nucleic acid. It is preferred that the endogenous nucleic acid is not an immunoglobulin gene or a part thereof. In this embodiment, only *DIVAC* is provided to the cell and it is secured that *DIVAC* integrates into the cell chromosome in the vicinity of the target nucleic acid.

According to the invention, the target nucleic acid is linked to *DIVAC.* This implies that the two nucleic acids are on the same chromosome in a position relative to each other that ensures that *DIVAC* activates and directs the diversification of the target nucleic acid. Generally, it is understood that the diversification activity of *DIVAC* diminishes with the increase in the distance to the nucleic acid to be diversified. It is therefore preferred that the distance between *DIVAC* and the target nucleic acid is no more than 150 kb, preferably no more than 125 kb, preferably less than 120 kb, less than 100 kb, less than 70 kb, less than 50 kb, less than 30 kb, less than 10 kb, or less than 5 kb.

According to the invention, the target nucleic acid and/or *DI*VAC may be integrated into the chromosome of a recipient cell at a defined or random location. In one embodiment, a genetic construct comprising an exogenous target nucleic acid and *DIVAC* is integrated into the cell chromosome at a random location.

In another embodiment, a first genetic construct comprising an exogenous target nucleic acid, i.e., a transgene, is integrated at a defined chromosomal position and a second genetic construct comprising *DIVAC* is integrated at a chromosomal position that ensures that *DIVAC* activates and directs diversification of the transgene. It is preferred that *DIVAC* is integrated in the vicinity of the transgene, preferably not farther than 150 kb away therefrom.

In a further embodiment, the target nucleic acid is endogenous, and a construct comprising *DIVAC* is integrated at a chromosomal position that ensures that *DIVAC* activates and directs diversification of the target nucleic acid. It is preferred that *DIVAC* is integrated in the vicinity of the target nucleic acid, preferably not farther than 150 kb away therefrom.

Integration at a defined chromosomal position (targeted integration) is achieved by including into the respective genetic construct nucleic acid fragments whose sequence is identical or homologous to the nucleic acid sequence at the integration site. The ability of a cell to integrate genetic constructs by targeted integration is an intrinsic property of the cell. Cells of different species and origins possess this property to a varying degree. For example, targeted integration is an infrequent event in a mouse cell. In contrast, the ALV-transduced chicken B cell line DT40 integrates genetic constructs preferably by targeted integration at a defined chromosomal position.

A recipient cell of the invention is a eukaryotic cell expressing activation-induced deaminase (AID) or a functional equivalent thereof. It is preferred that the recipient cell is a B lymphocyte from a vertebrate species such as a bird, a rabbit, a sheep, a cow, a pig, a mouse or a rat. It is further preferred that the recipient cell is a chicken B lymphocyte or derived thereof, in particular the ALV-transduced chicken B cell line DT40 or a derivative thereof.

A recombinant recipient cell of the invention is a recipient cell comprising a diversification activator of the invention and a target nucleic acid.

In one embodiment, the recombinant recipient cell expresses the target nucleic acid in a manner that facilitates selection of cells comprising mutants of said target nucleic acids having a desired activity. The selection may be a direct selection for the activity encoded or otherwise determined by the target nucleic acid within the cell, on the cell surface or outside the cell. Alternatively, the selection is an indirect selection for the activity encoded by a reporter nucleic acid or otherwise determined by a reporter.

According to the invention, diversification of the target nucleic acid may be modulated by a trans-acting regulatory factor. For example, the trans-acting regulatory factor may be DNA repair or recombination factor, a DNA polymerase, or an uracyl DNA glycosylase. The diversification process may also be initiated and terminated by a trans-acting regulatory factor such as activation-induced deaminase (AID). For example, AID may be conditionally expressed and its expression may be switched off once the targeted nucleic acid with a desired property is obtained. This would prevent further mutations that may result in a loss of the optimal property.

In another aspect, the invention provides a method for preparing a target nucleic acid having a desired activity, comprising the steps of:
(a) introducing into a recipient cell one or more nucleic acid construct(s) comprising a diversification activator of the invention and, optionally, the target nucleic acid to obtain a recombinant recipient cell,
(b) identifying a recombinant recipient cell, wherein said diversification activator is linked to said target nucleic acid,
(c) propagating and expanding the cell from (b) under conditions appropriate for the expression and diversification of the target nucleic acid, and
(d) selecting within the population of cells from (c) individual cells or cell populations containing a mutated target nucleic having a desired activity.

In one embodiment, steps (c) and (d) of the method are iteratively repeated.

In another embodiment, the method may include an additional step (e) comprising determining the sequence of the mutated target nucleic acid from the cells selected in (d).

In a further embodiment, the method additionally includes a step (f) comprising terminating the diversification. The diversification may, for example, be switched off by down-regulation of the expression of a trans-acting regulatory factor such as activation-induced deaminase (AID) or by the removal of the diversification activator.

In step (a), the nucleic acid constructs of the invention may be transfected into the recipient cell. Alternatively, the recipient cell may be transduced or infected with the construct.

In step (d), a variety of selection procedures may be applied for the isolation of mutants having a desired property. For example, cells expressing immunoglobulin molecules with improved or novel binding specificity may be selected by Fluorescence Activated Cell Sorting (FACS), cell separation using magnetic particles, antigen chromatography methods or other known cell separation techniques.

In one embodiment, the target nucleic acid and *DIVAC* are on the same nucleic acid construct. Alternatively, the target nucleic acid and *DIVAC* are on different constructs. In this embodiment, the genetic locus containing the target nucleic acid to be diversified may be constructed by more than one round of transfection. In a further embodiment, the target nucleic acid is part of the cell chromosome and the construct or constructs comprise (s) *DIVAC.*

In a third aspect, the invention provides a recombinant nucleic acid construct comprising a diversification activator of the invention. The construct may be a plasmid, a virus, a virus-derived vector such as an integrating or autonomously replicating vector. The construct may comprise one or more copies of *DIVAC*.

In a fourth aspect, the invention provides a recombinant cell comprising a diversification activator of the invention. Excluded form the scope of the invention are highly unlikely embodiment, wherein the diversification activator is integrated into the chromosome of a cell and replaced part of the chromosome identical to the diversification, such that the resulting cell is not distinguishable from a natural cell.

The invention is illustrated by the following examples.

### EXAMPLES

### Example 1: A Hypermutation Reporter Based on GFP expression

Inventors previously demonstrated that a *GFP* transgene in DT40 rapidly accumulated mutations, if integrated at the position of the promoter of the rearranged *IgL* [Arakawa et al., 2008]. The hypermutation activity depended on AID expression and could be visualized by the appearance of cells displaying decreased green fluorescence due to detrimental *GFP* mutations. To exploit this phenomenon a new expression cassette named *GFP2* was designed which consisted of the strong *RSV* promoter followed by the *GFP* coding region, an *internal ribosome entry site* (*IRES*), the *blasticidin resistance* gene (P19997) and the *Sv40* polyadenylation signal. *GFP2* was incorporated into the targeting construct *pIgL^{GFP2}* (Figure 1A) in the opposite transcriptional orientation of the *IgL* gene to minimize interference between transcription and post-transcriptional regulation of the *GFP2* transgene and the *IgL* gene.

Transfection of *pIgL^{GFP2}* into the conditionally AID expressing clone AID^{R2} yielded a number of transfectants named IgL^{GFP2} in which targeted integration had substituted the *IgL* promoter by the *GFP2* transgene. Fluorescence activated cell sorting (FACS) analysis of subclones from two independent primary transfectants revealed median values of 12.8% and 14.5% decreased green fluorescence (Figure 1C and 1D). The result confirms the results of inventors' previous study indicating that the *GFP2* transgene is mutated at high rate within the rearranged *IgL* locus and that cell populations with decreased green fluorescence can be used to quantify this hypermutation activity.

### Example 2: Hypermutation in the vicinity of the IgL locus

Targeted integration was used to insert the *GFP2* reporter at various distances from the *IgL* locus into chromosome 15 [International Chicken Genome Sequencing Consortium, 2004] (Figure 1B and 1E). FACS analysis of primary transfectants and their subclones revealed that the medians of decreased green fluorescence fell to about 3% at the +26 kb and the -15 kb positions, to 0.5% at the +52 kb position and to about 0.05% at the -135 kb position (Figure 1C and 1D). Although it could not be determined for the *GFP2* insertions outside the *IgL* locus, whether the rearranged or the unrearranged chromosome was targeted, the results of the subclones were also representative for a large number of independent primary transfectants (Table 1A). Thus, hypermutation of the *GFP2* reporter was detectable at insertions up to 52 kb away from the *IgL* locus, but incidence of mutations declined with increasing distance and were barely detectable at the -135 kb position.

Since surrounding sequences should not influence the post-transcriptional processing and translation of *GFP2* transcripts, *GFP2* transcription would be reflected by the green fluorescence of the cells independent of the transgene insertion site. Even for mutating transgenes, *GFP2* transcription levels could be deduced from the average green fluorescence of the major cell populations which most likely expressed the non-mutated *GFP* sequence. As seen by FACS analysis, the average green fluorescence of the major cell populations varied slightly among the primary transfectants (Figure 1C) most likely reflecting chromosomal position effects. However, the transfectants +52IgL^{GFP2} and IgL^{GFP2} differed more than 20 fold in their median fluorescence decreases despite similar green fluorescence of their major cell populations. This strongly suggested that the hypermutation differences among the transfectants reflected the distance of the *GFP2* insertion sites to the *IgL* locus and not variation in *GFP2* transcription.

### Example 3: Identification of a diversification activator

The effects observed in Examples 1 and 2 could be explained by the presence of a *cis*-acting sequence that activated hypermutation in a distance-dependent manner. This putative regulatory sequence was designated Diversification Activator *(DIVAC)*. Mapping of *DIVAG* was done by combining insertions of the *GFP2* reporter with deletions of the *IgL* locus.

To address the role of the ψ*V* locus, a *GFP2* construct (Figure 2A, upper part) was transfected into the clone ψV⁻AID^{R1} [Arakawa et al., 2008] in which the entire 20 kb of the ψ*V* locus had been deleted. The transfectants ψV⁻IgL^{GFP2} expressed the *GFP2* reporter at the position of the *IgL* promoter in the absence of the ψ*V* locus (Figure 2B). FACS analysis of ψV⁻IgL^{GFP2} subclones revealed medians of 5.2% and 7.5% decreased green fluorescence (Figure 2C), only one fold lower than the medians of the ψ*V* positive IgL^{GFP2} subclones. As the difference between IgL^{GFP2} and ψV⁻IgL^{GFP2} subclones could be due to fluctuation effects or different AID expression levels in the AID^{R2} and ψV⁻AID^{R1} precursors, the ψ*V* locus seems to exert little, if any stimulation on the hypermutation activity of the *GFP2* reporter. ψV⁻IgL^{GFP2} still contained a 9.8 kb fragment (referred to in the following as fragment *'W'*) of the rearranged *IgL* locus extending from the *IgL* transcription start site to the 3' end of the carbonic anhydrase gene. To test the relevance of this fragment, a *GFP2* construct was transfected into the clone ψV⁻IgL⁻ in which the entire rearranged *IgL* locus had been replaced by the *puromycin resistance* gene (P42670). The resulting transfectants ψV⁻IgL^{-,GFP2} had inserted the *GFP2* reporter at the position of the deleted *IgL* locus (Figure 2A, middle part and Figure 2B). Subclones of ψV⁻IgL^{-,GFP2} showed medians of 0.01% and 0.02% decreased green fluorescence (Figure 2C), more than 100 fold lower than the medians of ψV⁻IgL^{GFP2} subclones. This indicated that the '*W*' fragment, - absent in ψV⁻IgL^{-,GFP2} but present in ψV⁻IgL^{GFP2} -, was required for hypermutation of the *GFP2* transgene. ψV⁻IgL⁻ cells were then transfected by a construct including the *GFP2* transgene and the '*W*' fragment (Figures 2A, lower part). Subclones of the transfectants ψVIgL^{W,GFP2} showed median green fluorescence decreases similar to the medians of ψV⁻IgL^{GFP2} subclones (Figure 2C). Thus, the 'W' fragment efficiently activates hypermutation after reinsertion into the *IgL* locus as expected for a true *DIVAC* sequence.

Controls confirmed that the appearance of cells with decreased green fluorescence reflected hypermutation in the *GFP2* gene. As expected, the decrease of green fluorescence in ψV⁻IGL^{GFP2} cultures depended on AID, because subclones of the AID negative transfectant ψV⁻IgL^{GFP2}AID^{-/-} showed only very low medians of 0.001% decreased green fluorescence (Figure 2C). Furthermore, *GFP* open reading frames amplified from ψV⁻IgL^{GFP2} cells six weeks after subcloning showed 0.9 nucleotide substitutions on average (Figure 2D). The most prevalent mutations were C to G and G to C transversions as previously observed for hypermutation of the *IgL VJ* segments from ψ*V* deleted clones [Arakawa et al., 2004]. In contrast, only a very low number of nucleotide substitutions, most likely reflecting polymerase chain reaction (PCR) artifacts, were found in the *GFP* gene of ψV⁻IgL^{-,GFP2} cells (Figure 2D).

### Example 4: Fine mapping of DIVAC

A new series of targeting constructs was transfected into ψV⁻IgL⁻ to characterize the '*W*' fragment by step-wise deletions (Figure 3A). FACS analysis of subclones from the different transfectants showed a variable but progressive loss of hypermutation activity when the '*W*' fragment was shortened from either end (Figure 3C). The 4 kb '*S*' fragment in the middle of the '*W*' fragment which included the previously identified *IgL* enhancer [Bulfone_Paus et al., 1995] still produced median green fluorescence decreases of 2.7% and 1.7%. In contrast, the upstream '*B*' and the downstream '*P*' fragments on their own produced median green fluorescence decreases of about 0.13% and 0.05% respectively, which are low in absolute terms, but clearly above the medians of ψV⁻IgL^{-,GFP2}. If either one of these fragment was combined with the '*S*' fragment in the '*F*' and '*K*' fragments respectively, the median decreases of green fluorescence were elevated about 3 times. This suggested that the *DIVAC* of the chicken *IgL* locus consisted of a central core region and partially redundant flanking regions which contributed to the overall activity. More detailed analysis is being presently conducted to define the location, the nature and the configuration of the active motifs within the *IgL DIVAC*.

The average green fluorescence in the main population of ψV⁻IgL^{W,GFP2} was increased compared to ψV⁻IgL^{-,GFP2} (Figure 2B) perhaps due to the additional stimulation of the *RSV* promoter in *GFP2* by the *IgL* enhancer of the '*W*' fragment. However, the relatively small decrease of *GFP2* transcription seen in ψV⁻IgL^{-,GFP2} was unlikely to be responsible for the more than 300 fold reduction of hypermutation. Analysis of the '*W*' fragment deletions also strongly argued against the possibility that differences in hypermutation were caused by alterations of *GFP2* transcription since primary transfectants of all fragments shown in Fig. 3B showed similar *GFP* transcription levels.

### Example 4: Hypermutation at non-Ig Loci

To confirm that the *GFP2* reporter on its own is stably expressed at *non-Ig* loci, six loci on five different chromosomes [International Chicken Genome Sequencing Consortium, 2004] were targeted by transfection of *GFP2* constructs into ψV⁻AID^{R1} (Figure 4A and 1F). Neither the primary transfectants (Figure 4B, Table 1B) nor their subclones (Figure 4C) showed high percentages of decreased green fluorescence. Depending on the experiment and the insertion site, the medians of the subclones ranged from 0.02% to 0.22% indicating that the mutation rates of the *GFP2* reporter at the chosen loci were 50 to 500 fold lower than at the *IgL* locus. However, these medians were about 2 - 10 fold higher than the medians of various subclones from AID negative transfectants (Figure 2C and 5C) confirming a slight increase in the background mutation rates in AID expressing B cells [Liu et al., 2008].

*GFP2* was then inserted together with the '*W*' fragment into the respective *AID, BACH2* (NC_006090) and *RDM1* (BAC02561) loci of ψV⁻AID^{R1} (Figure 5A and 5B). Subclones of the transfectants ψV⁻AID^{W,GFP2}, ψV⁻BACH2^{W,GFP2} and ψV⁻RDM1^{W,GFP2} showed high median decreases of green fluorescence between 4.0% and 9.4% (Figure 5C) similar to the medians for ψV⁻IgL^{GFP2} and ψV⁻IgL^{R,GFP2} subclones. *GFP2* hypermutation was AID dependent, since subclones of the *AID* negative transfectants ψV-AID^{W,GFP2/-}, ψV⁻BACH2^{W,GFP2/-}AID^{-/-} and ψV-RDM1^{W,GFP2/-}AID^{-/-} showed very low medians of decreased green fluorescence in the range of 0.001% to 0.03%. These results demonstrated that the '*W*' fragment was able to activate *AID* mediated hypermutation at loci which otherwise did not support hypermutation.

### Example 5: Materials and Methods

Targeting constructs. The *GFP2* construct was made by combining the *RSV* promoter - *GFP* open reading frame of *pHypermut2* [20] with a PCR amplicon including an *IRES* [Arakawa et al., 2004], the *blasticidin resistance* gene and the *SV40* polyadenylation signal [Arakawa et al., 2001]. The PCR was performed using the primers described in the Supplementary table 2. *GFP2* was flanked by unique *BamHI* restriction sites for easy cloning into the targeting vectors.

All targeting constructs except the ones belonging to the series of '*W*' fragment deletions and reconstitutions were made by cloning the arms sequences into *pBluescriptKS*+ (Stratagene, CA) and then inserting *GFP2* either into unique *BamHI* or *BglII* sites as shown in Fig. 5A and Fig. 1E and 1F. Targeting of *AID* [Arakawa et al., 2001] and *RDM1* [Hamimes et al., 2004] have been previously described. The PCR amplifications of all target arms were performed using the Expand long template PCR System (Roche, Switzerland), DT40 genomic DNA as template and primers as described in Table 2.

Since the '*W*' fragment was difficult to amplify as a single sequence, it was sequentially cloned by combining upstream and downstream PCR amplicons with a 2.2 kb *AvrII*/*SpeI* restriction fragment excised from the rearranged *IgL* targeting construct '*Construct R*' [Buerstedde and Takeda, 1991]. The sequence of the *AvrII*/*SpeI* restriction fragment is A/T rich and localized between the J segment and the C region. The assembled '*W*' fragment was sequenced and deposited into Genbank under the accession number bankit1069926. Constructs belonging to '*W*' fragment deletion series were made by cloning *GFP2* between the target arms and then inserting the '*W*' fragment or parts thereof into unique *NheI*/*SpeI* sites. A *BamHI* fragment containing *GFP2* and the '*W*' fragment was incorporated into the *AID, BACH2* and RDM1 targeting vectors to test the activity of the '*W*' fragment in *non-Ig* loci.

Cell culture. Cells were cultured in chicken medium (RPMI-1640 or DMEM/F-12 with 10% fetal bovine serum, 1% chicken serum, 2 mM L-glutamine, 0.1 µM β-mercaptoethanol and penicillin/streptomycin) at 41°C with 5% CO2. Transfections were performed by electroporation, using 40 µg of linearized plasmid DNA with a Gene Pulser Xcell (BIO-RAD) at 25 µF and 700 V. Stable transfectants were selected by culturing in 15 µg/ml of blasticidin. Transfectants having integrated the transgenic constructs by targeted integration were identified by PCR using an inside primer from the *SV40* polyadenylation signal sequence of *GFP2* together with a primer derived from the sequence outside the target arm (Table 2). In case of insertions into the *IgL* locus, targeted integration into the rearranged allele was verified by amplifying the *VJ* intervening sequence of the unrearranged locus. The *AID* reconstituted clone AID^{R2} was generated from the *AID* deleted clone AID^{-/-} [Arakawa et al., 2002] by transfection of a construct which targeted an *AID* cDNA expression cassette into one of the deleted *AID* loci. The AID negative transfectants were produced by transfecting ψV⁻AID^{-/-} [Arakawa et al., 2004].

Flow cytometry. The phenotype of each mutation was determined by FACS analysis of at least two independent targeted transfectants and twenty-four subclones of each. The primary transfectants were analyzed by FACS about three weeks after transfection and the subclones two weeks after subcloning. As the green fluorescence levels in the main populations varied slightly among the transfectants, the gates to separate the main population of green fluorescent cells from cells showing decreased or lost green fluorescence were adapted accordingly.

At least 5000 events falling into the live cell gate were collected for each primary transfectant or subclone. Subclones in which more than 50% of the live cell events fell into the gates for decreased or lost green fluorescence were excluded from the analysis as they might represent the expansion of a precursor cell already expressing a mutated *GFP2* transgene at the time of subcloning.

*GFP* Gene Sequencing. To minimize PCR-introduced mutations, *Pfu* Ultra hotstart polymerase (Stratagene) was used for the amplifications of the *GFP* open reading frames prior to sequencing. Sequencing and sequence analysis were performed as previously described [Arakawa et al., 2004].

### REFERENCES

1. Muramatsu M, Kinoshita K, Fagarasan S, Yamada S, Shinkai Y, et al. (2000) Class switch recombination and hypermutation require activation-induced cytidine deaminase (AID), a potential RNA editing enzyme. Cell 102: 553-563.
2. Arakawa H, Hauschild J, Buerstedde JM (2002) Requirement of the activation-induced deaminase (AID) gene for immunoglobulin gene conversion. Science 295: 1301-1306.
3. Harris RS, Sale JE, Petersen-Mahrt SK, Neuberger MS (2002) AID is essential for immunoglobulin V gene conversion in a cultured B cell line. Curr Biol. 12 (5) :435-8.
4. Di Noia J, Neuberger MS (2002) Altering the pathway of immunoglobulin hypermutation by inhibiting uracil-DNA glycosylase. Nature 419: 43-48.
5. Rada C, Di Noia JM, Neuberger MS (2004) Mismatch recognition and uracil excision provide complementary paths to both Ig switching and the A/T-focused phase of somatic mutation. Mol Cell. 16(2):163-71.
6. Peters A, Storb U (1996) Somatic hypermutation of immunoglobulin genes is linked to transcription initiation. Immunity. 4(1):57-65.
7. Shinkura R, Tian M, Smith M, Chua K, Fujiwara Y, et al. (2003) The influence of transcriptional orientation on endogenous switch region function. Nat Immunol. 4(5):435-41.
8. Shen HM, Peters A, Baron B, Zhu X, Storb U (1998) Mutation of BCL-6 gene in normal B cells by the process of somatic hypermutation of Ig genes. Science. 280(5370):1750-2.
9. Liu M, Duke JL, Richter DJ, Vinuesa CG, Goodnow CC, et al. (2008) Two levels of protection for the B cell genome during somatic hypermutation. Nature. 451(7180):841-5.
10. Odegard VH, Schatz DG (2006) Targeting of somatic hypermutation. Nat Rev Immunol. 6(8):573-83.
11. Betz AG, Milstein C, González-Fernández A, Pannell R, Larson T, et al. (1994) Elements regulating somatic hypermutation of an immunoglobulin kappa gene: critical role for the intron enhancer/matrix attachment region. Cell. 77(2):239-48.
12. Klotz EL, Storb U (1996) Somatic hypermutation of a lambda 2 transgene under the control of the lambda enhancer or the heavy chain intron enhancer. J Immunol. 157(10):4458-63.
13. Klix N, Jolly CJ, Davies SL, Brüggemann M, Williams GT, et al. (1998) Multiple sequences from downstream of the J kappa cluster can combine to recruit somatic hypermutation to a heterologous, upstream mutation domain. Eur J Immunol. 28(1):317-26.
14. Kong Q, Zhao L, Subbaiah S, Maizels N (1998) A lambda 3' enhancer drives active and untemplated somatic hypermutation of a lambda 1 transgene. J Immunol. 161(1):294-301.
15. van der Stoep N, Gorman JR, Alt FW (1998) Reevaluation of 3'Ekappa function in stage- and lineage-specific rearrangement and somatic hypermutation. Immunity. 8(6):743-50.
16. Inlay MA, Gao HH, Odegard VH, Lin T, Schatz DG, et al. (2006) Roles of the Ig kappa light chain intronic and 3' enhancers in Igk somatic hypermutation. J Immunol. 177(2):1146-51.
17. Wang CL, Harper RA, Wabl M (2004) Genome-wide somatic hypermutation. Proc Natl Acad Sci U S A. 101(19):7352-6.
18. Yoshikawa K, Okazaki IM, Eto T, Kinoshita K, Muramatsu M, et al. (2002) AID enzyme-induced hypermutation in an actively transcribed gene in fibroblasts. Science. 296(5575):2033-6.
19. Arakawa H, Saribasak H, Buerstedde JM (2004) Activation-induced cytidine deaminase initiates immunoglobulin gene conversion and hypermutation by a common intermediate. PLoS Biol. 2: E179.
20. Arakawa H, Kudo H, Batrak V, Caldwell RB, Rieger MA, et al. (2008) Protein evolution by hypermutation and selection in the B cell line DT40. Nucleic Acids Res. 36(1):e1.
21. Gopal AR, Fugmann SD (2008) AID-mediated diversification within the IgL locus of chicken DT40 cells is restricted to the transcribed IgL gene. Mol Immunol. 45(7):2062-8.
22. International Chicken Genome Sequencing Consortium (2004) Sequence and comparative analysis of the chicken genome provide unique perspectives on vertebrate evolution. Nature 432: 695-716.
23. Bulfone-Paus S, Reiners-Schramm L, Lauster R (1995) The chicken immunoglobulin lambda light chain gene is transcriptionally controlled by a modularly organized enhancer and an octamer-dependent silencer. Nucleic Acids Res. 23(11):1997-2005.
24. Kothapalli N, Norton DD, Fugmann SD (2008) Cutting Edge: A cis-Acting DNA Element Targets AID-Mediated Sequence Diversification to the Chicken Ig Light Chain Gene Locus. J Immunol. 180(4):2019-23.
25. Arakawa H, Lodygin D, Buerstedde JM (2001) Mutant loxP vectors for selectable marker recycle and conditional knock-outs. BMC Biotechnol 1:7.
26. Hamimes S, Arakawa H, Stasiak AZ, Kierzek AM, Hirano S, et al. (2004) RDM1, a novel RNA recognition motif (RRM)-containing protein involved in the cell response to cisplatin in vertebrates. J Biol Chem. 280(10):9225-35.
27. Buerstedde JM, Takeda S (1991) Increased ratio of targeted to random integration after transfection of chicken B cell lines. Cell 67: 179-188.
28. Schoetz U, Cervelli M, Wang YD, Fiedler P, Buerstedde JM (2006) E2A expression stimulates Ig hypermutation. J Immunol. 177:395-400.

## Claims

1. Method for diversification of a target nucleic acid comprising introducing a genetic construct comprising a diversification activator (*DIVAC*) into a recipient cell to produce a recombinant recipient cell,
wherein said diversification activator is linked to the target nucleic acid within said recombinant recipient cell,
wherein said diversification activator is selected from the group consisting of a nucleic acid having the sequence SEQ ID NO:1, a fragment of said nucleic acid and a nucleic acid homologous to said nucleic acid and said fragment, and
wherein said diversification activator has the function of activating genetic diversification in transcription units linked thereto.

2. The method according to claim 1, wherein said diversification is somatic hypermutation.

3. The method according to claim 1, wherein the target nucleic acid is in addition linked to at least one nucleic acid capable of serving as a gene conversion donor for the target nucleic acid, and wherein said diversification is a combination of somatic hypermutation and gene conversion.

4. The method according to claims 1 to 3, wherein said diversification activator is a nucleic acid having the sequence between positions 1-7099, 1-7799, 2184-9784, 3098-9784, 4102-9784, 5114-9784, 6107-9784, or 3098-7099 of SEQ ID NO:1.

5. The method according to claims 1 to 4, wherein the length of said diversification activator is at least 20 nucleic acids.

6. The method according to claims 1 to 5, wherein said diversification activator shares at least 50% sequence identity with SEQ ID NO:1 of a fragment thereof.

7. The method according to claims 1 to 6, wherein more than one copy of said diversification activator is present in said recombinant recipient cell.

8. The method according to claims 1 to 7, wherein the target nucleic acid is an exogenous nucleic acid.

9. The method according to claims 1 to 8, wherein the target nucleic acid encodes an immunoglobulin chain, a selection marker, a DNA-binding protein, an enzyme, a receptor protein, or parts thereof.

10. The method according to claims 1 to 9, wherein the distance between the target nucleic acid and the diversification activator is no more than 150 kb.

11. The method according to claims 1 to 10, wherein said diversification of said target nucleic acid in the presence of said diversification activator is at least 5-fold higher than in the absence of said diversification activator.

12. The method according to claims 1 to 11, wherein the recipient cell is a eukaryotic cell expressing activation-induced deaminase (AID) or a functional equivalent thereof.

13. The method according to claims 1 to 12, wherein the recipient cell is a B lymphocyte or a cell line derived thereof, preferably the chicken B cell line DT40 or a derivative thereof.

14. Method for preparing a target nucleic acid having a desired activity, comprising the steps of:
(a) introducing into a recipient cell one or more nucleic acid construct(s) comprising a diversification activator and, optionally, the target nucleic acid to obtain a recombinant recipient cell,
(b) identifying a recombinant recipient cell, wherein said diversification activator is linked to said target nucleic acid,
(c) propagating and expanding the cell from (b) under conditions appropriate for the expression and diversification of said target nucleic acid, and
(d) selecting within the population of cells from (c) individual cells or cell populations containing a mutated target nucleic having a desired activity,
wherein said diversification activator is selected from the group consisting of a nucleic acid having the sequence SEQ ID NO:1, a fragment of said nucleic acid and a nucleic acid homologous to said nucleic acid and said fragment, and
wherein said diversification activator has the function of activating genetic diversification in transcription units linked thereto.

15. The method according to claim 14, wherein steps (c) and (d) are iteratively repeated.

16. The method according to claims 14 and 15, further including (e) determining the mutant sequence of the target nucleic acid from the cells selected in (d).

17. The method according to claims 14 to 16, further including (f) terminating said diversification.

18. A recombinant nucleic acid construct comprising a diversification activator, wherein said diversification activator is selected from the group consisting of a nucleic acid having the sequence SEQ ID NO:1, a fragment of said nucleic acid and a nucleic acid homologous to said nucleic acid and said fragment, and wherein said diversification activator has the function of activating genetic diversification in transcription units linked thereto.

19. A recombinant cell comprising a diversification activator,
wherein said diversification activator is selected from the group consisting of a nucleic acid having the sequence SEQ ID NO:1, a fragment of said nucleic acid and a nucleic acid homologous to said nucleic acid and said fragment, and wherein said diversification activator has the function of activating genetic diversification in transcription units linked thereto.


SEQUENCE LISTING

<110>  Helmholtz Zentrum München Deutsches Forschungszentrum für
       Gesundheit und Umwelt, GmbH

<120>  Cis-acting Diversification Activator and Method for Selective
       Diversification of Nucleic Acids

<130>  P 77183

<160>  107

<170>  PatentIn version 3.4

<210>  1
<211>  9784
<212>  DNA
<213>  Gallus gallus

<400>  1
ttccgccatg gcctgggctc ctctcctcct ggcggtgctc gcccacacct caggtactcg      60

ttgcgcccgg tcggggactg tgggcacggg gctctgtccc actgctgcgc gggcagggct     120

gtgcgtgcgg ggccgtcact gattgccgtt ttctcccctc tctcctctcc ctctccaggt     180

tccctggtgc aggcagcgct gactcagccg gcctcggtgt cagcaaatcc aggagaaacc     240

gtcaagatca cctgctccgg gggtggcagc tatgctggaa gttactatta tggctggtac     300

cagcagaagt ctcctggcag tgcccctgtc actgtgatct atgacaacga caagagaccc     360

tcggacatcc cttcacgatt ttccggttcc aaatccggct ccacagccac attaaccatc     420

actgggttcc gagccgatga cgaggctgtc tatttctgtg ggagctacga agacaacagt     480

ggtgctgcat ttggggccgg gacaaccctg accgtcctag gtgagtcgct gacctcgtct     540

cggtctttct tcccccatcg tgaaattgtg acattttgtc gatttttggt gatttggggg     600

tttttcttgg acttggcggc aggctggggt ctgccaccgg cgcagggccg ggcactcagc     660

gcggcagcct gggctgagtc ttgtccccac cgagccggag ggctccggtg tgcgccatgg     720

aggacttagg gttattttgt caatggaaag ttcttaaaat ttgaccagaa aatgtgcccg     780

aggtctgtct ctgccacacg atttcagaaa ttgtgtctag gtcgatgaga agacagtttt     840

tgtctttgtc aggaaattag ttgtgagttg ttagtccttc cctcttagtc ctaaggacta     900

agacctttgt ccccggtctg gtctctcact ggggactctt ggctccagtg ccatggggag     960

cccaagtgtc actgacacgg tgtccttggg ggtgaaattc agtttttcag ctgtccaggt    1020

aagtgctgtc aaaaaatgga ggttattctg ctgaaaaagc tgagaggaat attttgtcat    1080

ttttttcgga aatatatata tatatatata tataaatata taaattattt atatatttta    1140

tatatatata tatataaaat atatgtattt ctctctttct ttttatatat atatataaat    1200

atatatatat ttctccctct ttctttatat atatttatag ggagagaatc tatatttttg    1260

gccaatttgg ccaatttctc tctctctctc tttatatata tatatatata tatatgtaat    1320

ttatatatat atatataaat ttatatttat ttatttattt atttatatag attattttta    1380

tatacatata tatatataaa tttggccaat ttagaagaaa ttggccaaaa aaacccaatc    1440

tgaaccaaat tacttagggc ataagtccaa aaaaaaagtg cagttcaaga attcctcgct 1500
ggaaagtttt taatgggggc agttcttccc cattttctgg tgtagatatg agtgaagata 1560
tagatatgta tgtagatata gatttggcta tagtttgttt gatgaacagt ctgaggaaga 1620
gtaaatcttg ccttcctcat ggcaattttt aattctactt cttctgagac aagtctgaac 1680
atgccttgag gtagaaagag cccgaaaatt gagtgctttt tctttgaaat tttaattaaa 1740
attaaaaaat agccttacag caggaaagaa agggggtgat ggcagctaat cggagttcca 1800
aagctgaacc acgttcaccc aaacacgtgg ttgaaatttt gtgtgtgtat atatataaat 1860
atatatatgt atacaaaaga aaatgcctat atataaaaaa attgttatgt atattatata 1920
gatttattat agatatgtac ataagatata acacgtatac atatatatgt agtctgctgt 1980
atgctttgta taaattttta tagatatgat gtatattata tgcatgggtt atatatctat 2040
aatatatttt atctgtagaa ggaactacag tataaaatgc atatatgcgt atatatacac 2100
atatttatat atatgtgtat atatatgtat acgcacacgt ataacatcca tgaaaataga 2160
tatggatggg tggatgtgtt tgttttacag aggtgcatgt gtgtctgtac atacacagcc 2220
atacatacgc gtgtggccgc tctgcctctc tcttgcaggc cagcccaagg tggcccccac 2280
catcaccctc ttcccaccgt caaaggagga gctgaacgaa gccaccaagg ccaccctggt 2340
gtgcctgata aacgacttct accccagccc agtgactgtg gattgggtga tcgatggctc 2400
cacccgctct ggcgagacca cagcaccaca gcggcagagc aacagccagt atatggccag 2460
cagctacctg tcactgtctg ccagcgactg gtcaagccac gagacctaca cctgcagggt 2520
cacacacaac ggcacctcta tcacgaagac cctgaagagg tccgagtgct aatagtccca 2580
ctggggatgc aatgtgagga cagtggttcc tcaccctccc tgtccctctg ggccgctgct 2640
ggtggcagca gccctcactt cccactcaga tgtcccccac cgtgccccca tcacccacct 2700
ctgcctgtcg actcctcttg ccctcatctc tccaggtgtc acattaataa acacgacact 2760
gaactagtgc tgactctgca tccatgtctc tgtgtccttt tgcgtgctgt ctgcatctca 2820
cacagtgggg tcggccccag tatggggaag ggctgggggg cacatacaca catattggta 2880
atgttggggg cggggggggg agggcggggg ggtcaacaga tcagcactgg agacactggt 2940
gtataccctg gcaacaccaa catctaaggc agggtgcttt ggggcaattt tggggcagtt 3000
taaggtctgt gctggcactg agcacatggc tgtggccgtg ctgtcctcgt ctcccaccca 3060
ctacggtctg tgcgccaggt ccctagcaga gatttgcttt atgctgggaa cagggggagt 3120
tctgggtctg ttcccttgca ttcagacacc ctggtgcccc ctgggtggga tgtcagtgtg 3180
aatactcctt tgtgccctgt gcctgcagca gcctgaccct ccacacacca cacgccttgt 3240
gtgcacccca cccctgtcac tatccctctc cccgctcccc agggagattt tgcagtggcc 3300
cctgtagggc agcttttagc acagccccca gcagcaagca agcagaaagc actgctgtgc 3360
acagcttgtc agctgtgtgt gtttgctgag gaggatctgt cttttgctga ggccatcagt 3420
cttgtcctgc tcaacctcca tcgatgctgc ccacctcaac acatctaccc atctattcca 3480

tctacaccaa catctccatt catcccaccc acccaaacat gtccatccat cacaacacct 3540
ccatccaacc cgcacactcc agcacctcca atcattccat ctacaccacc atgctgatct 3600
gctacagcca ctccaacgca accgtccatt ccatctacac caatgtccat ccatcccagc 3660
cactccagca cctccagcca tcccacccac cctatgtctc catccagcca ctggtggggt 3720
gcaggacatg gggccagctc tactgtcagg actggggttt ttgcatggcc ccataccact 3780
tctgcagaag agacgcactg aaagtttggc tgaccatttt ctccgcggta gagctgtgga 3840
aattctgtaa tttagggtct tttatccagt ttggagatgg gctgggatct cccagctcca 3900
tggcaggcat tcatgacact gggtttagta tctgatgggt gggatgtggc tgaacttcat 3960
tttctttccc cagtgacaaa gtttttgcag ttgaatatga attcctgctt tctgctctat 4020
gagttgtttt tttcccagga cgtacacagg gaatcagcag tcttcattct ccctctgcca 4080
tgtgtagact ctctgccaca caggactgtg ctgtcctcat gcccctgcgc ccaaattgct 4140
gccctctgcc catgcctgcc aagctgagcc cccctgcagg ctgccatgct ggattgacat 4200
gagccctgag attggtacag aaatggtgat tttggggttt tctctgcact caggaagctg 4260
aaggctcaat gctcagtgat ggatttacca aactgtgccc tgaggcagct gctcatgctg 4320
gataaagtca ctggagcaca gggaaccagg cgctgggcag ggatttctca tgggccccac 4380
ttggaaagct gcaggctgca aacctggctc tgccttcacg cctcaccctc atgaggacaa 4440
cctcactaat tattgattaa aagattttgc taaaccatct ccagaagcaa caacccactg 4500
aggagcatgt gctgaattat acatcacagc tccacggccc tgccctcata gcagggctgc 4560
atggcaccca cagtggcact cagcgggacc acagggctga gacagccggg tctggtggtg 4620
gggacacagc tgagcatagg atgagccccc cgggcagtgc tgggctttgc taatgagcag 4680
aagtatggat agaaagcaac cccagggctc cggtcccagc tgcagctctt gctctgtcgt 4740
gtcctttggt gaaactttaa acagtcgcct ttttttttct ctttcttttc tggcttgcca 4800
ttaatttcaa accgagagag acctaattta gtaaatgaga tgcttcagga aggctttaat 4860
tggctgcaga tggaggcagg cagtgctatc gtggggcctg gatcgcacag ggggctgcat 4920
atcctcacta gcagaataca ctcaggctgg gtccctccca cattcatgcc ccagaccaga 4980
gggaatatgc tctgttcccc acacatctct cccaatcttg cagctgttga gccccaacat 5040
cccaccagca cacggggctc agcacgcctg gcgacgtggc atcagcagag caggccgcat 5100
ggtacagctc catcagcaca gctggggcca cacaaagagc tgggttactg tgggcagcag 5160
gctgaaaccc gaaaacaaga gctgggggct cagaatagcc ccgggagcag gcagggcctg 5220
ggggagaggg caagcaccag gcccagggcc acacagccct tccaggaagg cacagcgctg 5280
tcagggtgca gcacgctcag ccccaccatg cagctgtgcg gccggggcat ccccaagcta 5340
aatttacttc tcagtctcca atcagaaact gaagctgagg ggcccacgcc ggccaaaaaa 5400
aggaaacgaa acagtctcca gaaagcactg acgtgtgaag cagagcgagc gccgcgcaaa 5460
ccggccgcca tgtcacacac ctcaggttgg ggctttgcca gactgagctt tgctgctgct 5520

cggggtgggt gcccacggcc tgggcacatg ggatggggta cacacgtaca cacacttgca 5580
cacccacacc ccaacacttc aggtgatgct ggtgcagatg ggtgcccccc aggctgaccc 5640
ccccacgcat gggcctggcc ccacactgct ccatccgtgt ctctgtcccc atgtgccacc 5700
cctgcccgct cccaccacgc gtcaacccaa atcctgagtt aatcccacga ctcctgcctg 5760
cttccagcgt ccatggcaga ctggagatgc ccaaaatgca gagcaggttt ccctgaatct 5820
gagagatgaa atggagttat gggtgttccc ctgcggcgga gccccagctg taggaagctc 5880
agagccatca cacagcaatt aaagaggaat taaattaaat caataaatgt tttaggcggg 5940
ctcagctgcc agcaccacct gaccgaaaca gcccgcttgc aaagaggaga gcatttgcat 6000
ggctgtggca aaacagcaac cgcctgttgt gcagctggga tggtgttatc tggaaatgta 6060
cgcagcccag gaggggtaaa cagctccaaa ctgagacccc gagcttgtcc acaggttgta 6120
aacaggctga cataaacacc tttgtgccgt ggaaaaatat ttatcacctc aaatatagca 6180
ggttaataaa ataaaactcc caacggagct acacacctgc tttggaaggg aagcagacac 6240
ttgttttctg cttgatgttg gctgtaggaa gccatgtttc ccgatgcagg agggccacaa 6300
agcactgaca acacaatgtg agctgagctt cgcccctgtt taagccccca cctcagggct 6360
tgtggcctcg gagcaggcag gacgcagggg tggcaccggg ctgggtgaca tgggctggtc 6420
ctggggtgtc tcactgtgct ctttggggag gggttggagc cctggggcaa tcacagcaca 6480
cacaggaggt ggggggatgc agccagcagc tgccctgcac taagaaaacc ccatccgtgg 6540
gctttcagat ggccttccca tctctctgca gcctctgcat gggctgagca caaggtttaa 6600
gtgtttctgc catgtttttg ggcatgtttg gaggggcagc atgggcccgg gcatacgggt 6660
accgccacgt gccgccagcc ccacagctga gcctgcactc tcccagatgt gctgaccgca 6720
gccacggggg caacagtttc tcttgctaaa aattgtagcc gggaagaaaa cacgtggcaa 6780
cttcggccaa acagcagctg gaggacagga atagccgtgg ccacggcacg ctctgcttcc 6840
tcggcacaaa cattccagta tgtggcacca cgagcgccgc tgcccggcac agcagcaagc 6900
agagccagga gcaggaaatg ctgatttggg ccccattttg gccatggctg agagaagagg 6960
cttccaggga gctggtcagc ttggtcccca agctgtggct tggggaaatg atggggaggg 7020
gattgccact gcccaccctg cagagcaggc tctggtccca tctcactgca gggcaccagg 7080
gcgtttgcac tgcagcaatt cacagaaaca ttgaaatggc tcctgccttg ttcaacatct 7140
tcatcagtga cctggatgag gggacagcat ccaccatcag cgggttcact gatcatatga 7200
agtcgggagg agtggctgac gcaccacaag gctgtgctgc cattcaacag gacgtggaca 7260
gactggagag ctggacaggg aggaacccaa tgaggttcaa caatggcaag tgtaggatct 7320
acacctggga aggaataaca gcatgcatca gttcaggtta ggggctgagc tgctgcagat 7380
gagctctgag agaaggacct gagcatcctg ctggacagca ggctggctgt gagccaccgg 7440
tgtgccctgg tggccaagaa ggccagtggt atcctgggga gcaccgcaat gagagtgggc 7500
agcagggcga gggaggtgag gctgcatttg gagcaccgtg cccagttctg ggctcctcag 7560

ttcaaggcag acagggaact gctggagaga gcccagcaga ggggctgcaa tgatgatgag 7620
ggtcctggag catcgcctgt atgaggaaag gctgagggac ctgggattgt tcagcttgga 7680
gaagagaaga ctacagggca ggagccaagt ggatagggcc gggctctttt cagcagtgcc 7740
cagtgacagg acaaggggca gcaggcacaa agtggaacat aagaagttcc atctgaacat 7800
gaggaaaaac tgcctcgctt tgagggtgac cgagcactgg aagaagctgc ccagagaggt 7860
ggtggagtct cctctggaga tattcagagc ctggcaggac actttttgct gagtaaccta 7920
ctgtagggaa cctgacgcag cagaggggtc ggactggagg atctccggag gtctctttca 7980
acccctacag ttccatgaaa tacctcaaac actgccaagc gcagtgctaa ggcaagggta 8040
acatttgtaa actgaaacag ggtgggttta agttagatgt aaagaagaaa ctcttcactc 8100
agagggtggc gaggccctgg cacaggctgc ccatggaggc tgcgggtgcc ccatccctgg 8160
cagtgcccaa ggcaagagcc cagcagtgac cacagcccca caaggacgag cgtggccctc 8220
gtatctcagc tcaccctgcc ccagctcaac tcccacctcc ggcacagcgc gggcacacag 8280
ccgggccctg tgcttatgga gcccttgggg caggtcagca ctcacaccct ccaaacacag 8340
ccgtggctcc caaccggagg cagctggatc tcggcagcca taaccaagca gggccatgcg 8400
ggggtgacac cggggtcccc caccccctgt ggggcagcgt atgggctggg cccctgctcc 8460
agtctgcagc gtgtgcatgg gaaccattgc cagacaccgt ctggaccacc cgcagcccta 8520
agctgcctca cagcagggat tgctccgtca caccgtgacc ccgtgccctt attccatcac 8580
ttatggggct gggagtgcct ggaccttggg cacattaacg aggatttccc gctctgccct 8640
cgctttgctc cgagccgtgg ggctgtgtag tgcagacaca gctgcagcct aaaattagta 8700
cctgggaaag gcccccatgc tgcaccgcgc agggctgaga tgtgccacgt ccccatggcc 8760
ggagctgggg aaggcaacgt ggccctgtgc gtgtgcacgc tgagcacaag gacacgtgct 8820
gggccaggat ttgtctcccc ggggctcacg ctatgtgtca cccggtgctg cgccatcccc 8880
tcccgcagcc cccagctccc ccacggccgc acgccgcctg catccctgca acggcaccgc 8940
acagagacac ggagccaggg gccgcacacg gggccaggag ctcaccttta ttgcagccct 9000
gacagcccca cggcccagcc cgcaccgggg ctgccacatc ctcacccggc cgacggcccc 9060
cagctgctcc ttaccatttc ttccccccat caccccataa accagaagcc gcctcaccgc 9120
tacgcggagc gggcagcagg gaacccgggc cctaaggggg agacgagagg gggccgagca 9180
ggggcaggag gagcagcagg gcgagggggc agcgggggca cccacagctg gccgtggcat 9240
cccgggagga gaagaccttg cggctgcgga gcggttgtgg cggacggaaa ttgttggtca 9300
tcttcagggg cgcagcgccc gaggccggga agtgcacggt gctgacaaac gcctgcagct 9360
gcggggagag caccgcgggc gccgcagccg tgaggcgtag ggcgaagcgg ggcacacgcg 9420
tggctgctgc tgtctttccc cctaccggca gcacacggct ctgcacacac cgcgcttcgt 9480
gccgcctcgc agccgacgct gcaggaagcc cagccgagcg cttacagagc ggccgggaaa 9540
tgcatctgct gaggtgcccg ggcaatgcag aacttcatcc atccccacat ccattcacca 9600

gtcccctccc aaacccccaa gcccatccgg cgacccaccc accctcctct tggtgcccct 9660

ctcaagctct ccatccccac attcctacag atgtcccctt tactttgcct gcaaggtgca 9720

agaaaacgca caggaccggg ggtgctcaca gcacggcttt ggccagacgg gcccttccat 9780

ccca 9784

<210> 2
<211> 34
<212> DNA
<213> Gallus gallus

<400> 2
ggggtcgaca tatgatacac agacctgaca tctc 34

<210> 3
<211> 40
<212> DNA
<213> Gallus gallus

<400> 3
gggggatcca ctagtatcac ataatcacca aggttggaaa 40

<210> 4
<211> 34
<212> DNA
<213> Gallus gallus

<400> 4
gggggatcct tccgccatgg cctgggctcc tctc 34

<210> 5
<211> 34
<212> DNA
<213> Gallus gallus

<400> 5
ggggctagcc ctctcagctt tttcagcaga ataa 34

<210> 6
<211> 34
<212> DNA
<213> Gallus gallus

<400> 6
gggactagta aaatgatgca taaccttttg caca 34

<210> 7
<211> 30
<212> DNA
<213> Gallus gallus

<400> 7
cccaccgact ctagaggatc ataatcagcc 30

<210> 8
<211> 39
<212> DNA
<213> Gallus gallus

<400>  8
gggctcgagg gtactgcgtt ttccacaaaa ttctcacag                              39


<210>  9
<211>  39
<212>  DNA
<213>  Gallus gallus

<400>  9
gggagatctc tgcactctgg caccgttaag caccatcac                              39


<210>  10
<211>  38
<212>  DNA
<213>  Gallus gallus

<400>  10
gggtgatcaa gatctgctag cactagtgga tccgtcga                               38


<210>  11
<211>  39
<212>  DNA
<213>  Gallus gallus

<400>  11
ggggaaaagc ggccgccact ggaaggagct gaaggccac                              39


<210>  12
<211>  27
<212>  DNA
<213>  Gallus gallus

<400>  12
agcttggaat ttaacctctc ctgtaaa                                           27


<210>  13
<211>  30
<212>  DNA
<213>  Gallus gallus

<400>  13
cccaccgact ctagaggatc ataatcagcc                                        30


<210>  14
<211>  34
<212>  DNA
<213>  Gallus gallus

<400>  14
gggatcgatt tccctggtgt gggttttttt gggt                                   34


<210>  15
<211>  34
<212>  DNA
<213>  Gallus gallus

<400>  15
gggatcgatt tgagaaagct cacgctcctt ccaa                                   34


<210>  16

<211>  34
<212>  DNA
<213>  Gallus gallus

<400>  16
gggactagtc atctcagcgg tgcttatgaa tgac                                34


<210>  17
<211>  34
<212>  DNA
<213>  Gallus gallus

<400>  17
gggactagta ccccaaaaag cagccgagca tttc                                34


<210>  18
<211>  30
<212>  DNA
<213>  Gallus gallus

<400>  18
gctgctcacc ttccttaccc tgctgctcct                                     30


<210>  19
<211>  30
<212>  DNA
<213>  Gallus gallus

<400>  19
cccaccgact ctagaggatc ataatcagcc                                     30


<210>  20
<211>  34
<212>  DNA
<213>  Gallus gallus

<400>  20
tttatcgatg gagaagtgag cggcagaggg aaat                                34


<210>  21
<211>  34
<212>  DNA
<213>  Gallus gallus

<400>  21
tttatcgatt cagcagggca atgggcacac actg                                34


<210>  22
<211>  34
<212>  DNA
<213>  Gallus gallus

<400>  22
tttactagtg cgcagcagag cgcaccaacc acag                                34


<210>  23
<211>  34
<212>  DNA
<213>  Gallus gallus

<400>  23

gggactagtt cgacagcgag tgcaaaatca tctt 34

<210> 24
<211> 28
<212> DNA
<213> Gallus gallus

<400> 24
ggaccggggg tgctcacagc acggcttt 28

<210> 25
<211> 30
<212> DNA
<213> Gallus gallus

<400> 25
cccaccgact ctagaggatc ataatcagcc 30

<210> 26
<211> 33
<212> DNA
<213> Gallus gallus

<400> 26
gggactagtt gcccttttgc ttgcagccag cct 33

<210> 27
<211> 34
<212> DNA
<213> Gallus gallus

<400> 27
gggctcgaga atctcaacag ctgtgaagtt tcga 34

<210> 28
<211> 28
<212> DNA
<213> Gallus gallus

<400> 28
ggggaaacag tgagcatggg gattccct 28

<210> 29
<211> 30
<212> DNA
<213> Gallus gallus

<400> 29
cccaccgact ctagaggatc ataatcagcc 30

<210> 30
<211> 33
<212> DNA
<213> Gallus gallus

<400> 30
gggtctagaa gtagtccaac caacctatgc agt 33

<210> 31
<211> 33

<212>  DNA
<213>  Gallus gallus

<400>  31
gggctcgagt ctgaagcctg aaatcacaca gca                                33

<210>  32
<211>  25
<212>  DNA
<213>  Gallus gallus

<400>  32
ctttctatcc gtgcttagtc tggtt                                         25

<210>  33
<211>  30
<212>  DNA
<213>  Gallus gallus

<400>  33
cccaccgact ctagaggatc ataatcagcc                                    30

<210>  34
<211>  33
<212>  DNA
<213>  Gallus gallus

<400>  34
gggctcgagt agtctgtgca tgaaaatgtg ctg                                33

<210>  35
<211>  34
<212>  DNA
<213>  Gallus gallus

<400>  35
gggggatcca gcaattaacc aaatcctctg acag                               34

<210>  36
<211>  34
<212>  DNA
<213>  Gallus gallus

<400>  36
gggggatcct cgctccttta ttctctccag tggc                               34

<210>  37
<211>  34
<212>  DNA
<213>  Gallus gallus

<400>  37
gggtctagac agccagccta tgcactgcct ccac                               34

<210>  38
<211>  25
<212>  DNA
<213>  Gallus gallus

<400>  38
gtaacactga taagagagag atcag                                         25

<210>  39
<211>  30
<212>  DNA
<213>  Gallus gallus

<400>  39
cccaccgact ctagaggatc ataatcagcc                                        30


<210>  40
<211>  39
<212>  DNA
<213>  Gallus gallus

<400>  40
gggctcgagg tcatctgaga gagaacccag ctgacatgg                              39


<210>  41
<211>  38
<212>  DNA
<213>  Gallus gallus

<400>  41
gggggatccg cttcacaact taacagaggt aggtttca                               38


<210>  42
<211>  38
<212>  DNA
<213>  Gallus gallus

<400>  42
gggggatccg tgagagtact gaactgagtc ctggacag                               38


<210>  43
<211>  39
<212>  DNA
<213>  Gallus gallus

<400>  43
gggactagtc agtcaacatc aggcaggaag atctggttt                              39


<210>  44
<211>  30
<212>  DNA
<213>  Gallus gallus

<400>  44
gagcctgtga ggcaacttct gtgcaaccca                                        30


<210>  45
<211>  30
<212>  DNA
<213>  Gallus gallus

<400>  45
cccaccgact ctagaggatc ataatcagcc                                        30


<210>  46
<211>  34
<212>  DNA

<213>  Gallus gallus

<400>  46
gaactcgagt gcctgcgggg agcgcgcaga catt                                    34


<210>  47
<211>  41
<212>  DNA
<213>  Gallus gallus

<400>  47
gggggatcct tagtagaact tgatgatggc atagcagcca g                            41


<210>  48
<211>  34
<212>  DNA
<213>  Gallus gallus

<400>  48
gggggatcct ctggagtttg gcacagccac aaga                                    34


<210>  49
<211>  39
<212>  DNA
<213>  Gallus gallus

<400>  49
ggggctagcc atatgaggta gatgtcattg cacagcttt                               39


<210>  50
<211>  39
<212>  DNA
<213>  Gallus gallus

<400>  50
gaaagatcta ggggggcccg ggcatggcgg aggtgttgg                                39


<210>  51
<211>  30
<212>  DNA
<213>  Gallus gallus

<400>  51
cccaccgact ctagaggatc ataatcagcc                                         30


<210>  52
<211>  39
<212>  DNA
<213>  Gallus gallus

<400>  52
ggggaattca tatcctccgc tgtctcattg acatacatt                               39


<210>  53
<211>  39
<212>  DNA
<213>  Gallus gallus

<400>  53
gggggatcct ttgctgtata ttaatacttg atggaaaca                               39

<210> 54
<211> 39
<212> DNA
<213> Gallus gallus

<400> 54
gggggatccc aagcagttaa taagcttcca cgtcagatg 39

<210> 55
<211> 39
<212> DNA
<213> Gallus gallus

<400> 55
gggtctagag tcgcagtttc cgctgatacg tggcatcac 39

<210> 56
<211> 30
<212> DNA
<213> Gallus gallus

<400> 56
cttgccaagg gtacagctag catccctctt 30

<210> 57
<211> 30
<212> DNA
<213> Gallus gallus

<400> 57
cccaccgact ctagaggatc ataatcagcc 30

<210> 58
<211> 40
<212> DNA
<213> Gallus gallus

<400> 58
gggctcgagg atgactttca atatgcagat catgactacg 40

<210> 59
<211> 35
<212> DNA
<213> Gallus gallus

<400> 59
gggggatccc tctgatcttc ttatttagtc caagg 35

<210> 60
<211> 45
<212> DNA
<213> Gallus gallus

<400> 60
ggggtcgacg gatccaatta agaagaaact gaatgcgtgt tcttc 45

<210> 61
<211> 35
<212> DNA
<213> Gallus gallus

<400>  61
ggggtcgacg ctagctcaca gtatcagagc ccttg                                35

<210>  62
<211>  30
<212>  DNA
<213>  Gallus gallus

<400>  62
gagaccgcgc acgagcgaag aagatgatga                                      30

<210>  63
<211>  30
<212>  DNA
<213>  Gallus gallus

<400>  63
cccaccgact ctagaggatc ataatcagcc                                      30

<210>  64
<211>  34
<212>  DNA
<213>  Gallus gallus

<400>  64
gggatcgatt ccttggcata cgcccatggt ttgg                                 34

<210>  65
<211>  37
<212>  DNA
<213>  Gallus gallus

<400>  65
gggatcgatt caggccatct tgagtctgcc caggacg                              37

<210>  66
<211>  34
<212>  DNA
<213>  Gallus gallus

<400>  66
tttactagtg gcagcctgag ctggtggggg caag                                 34

<210>  67
<211>  34
<212>  DNA
<213>  Gallus gallus

<400>  67
gggactagtg gaattctgaa gaatcattct ggtg                                 34

<210>  68
<211>  30
<212>  DNA
<213>  Gallus gallus

<400>  68
gagctgtctc tgaaagaagc ggtgagaaaa                                      30

<210>  69
<211>  30
<212>  DNA
<213>  Gallus gallus

<400>  69
cccaccgact ctagaggatc ataatcagcc                                        30


<210>  70
<211>  39
<212>  DNA
<213>  Gallus gallus

<400>  70
gggctcgagg gtactgcgtt ttccacaaaa ttctcacag                              39


<210>  71
<211>  39
<212>  DNA
<213>  Gallus gallus

<400>  71
gggagatctc tgcactctgg caccgttaag caccatcac                              39


<210>  72
<211>  38
<212>  DNA
<213>  Gallus gallus

<400>  72
gggtgatcaa gatctgctag cactagtgga tccgtcga                               38


<210>  73
<211>  39
<212>  DNA
<213>  Gallus gallus

<400>  73
ggggaaaagc ggccgccact ggaaggagct gaaggccac                              39


<210>  74
<211>  27
<212>  DNA
<213>  Gallus gallus

<400>  74
agcttggaat ttaacctctc ctgtaaa                                           27


<210>  75
<211>  30
<212>  DNA
<213>  Gallus gallus

<400>  75
cccaccgact ctagaggatc ataatcagcc                                        30


<210>  76
<211>  35
<212>  DNA
<213>  Gallus gallus

<400> 76
gaagctagct tccgccatgg cctgggctcc tctcc 35

<210> 77
<211> 49
<212> DNA
<213> Gallus gallus

<400> 77
gaaactagta ttttttgaca gcacttacct ggacagctga aaaactgaa 49

<210> 78
<211> 34
<212> DNA
<213> Gallus gallus

<400> 78
ggggctagcg gtggatgtgt ttgttttaca gagg 34

<210> 79
<211> 34
<212> DNA
<213> Gallus gallus

<400> 79
gaagctagcg caaatctctg ctagggacct ggcg 34

<210> 80
<211> 34
<212> DNA
<213> Gallus gallus

<400> 80
ggggctagcg gtggatgtgt ttgttttaca gagg 34

<210> 81
<211> 34
<212> DNA
<213> Gallus gallus

<400> 81
gaagctagcg tgtggcagag agtctacaca tggc 34

<210> 82
<211> 34
<212> DNA
<213> Gallus gallus

<400> 82
ggggctagcg gtggatgtgt ttgttttaca gagg 34

<210> 83
<211> 34
<212> DNA
<213> Gallus gallus

<400> 83
gaagctagca tggagctgta ccatgcggcc tgct 34

<210> 84

<211>  34
<212>  DNA
<213>  Gallus gallus

<400>  84
ggggctagcg gtggatgtgt ttgttttaca gagg                                34


<210>  85
<211>  34
<212>  DNA
<213>  Gallus gallus

<400>  85
gaagctagca agctcagggt ctcagtttgg agct                                34


<210>  86
<211>  34
<212>  DNA
<213>  Gallus gallus

<400>  86
ggggctagcg gtggatgtgt ttgttttaca gagg                                34


<210>  87
<211>  34
<212>  DNA
<213>  Gallus gallus

<400>  87
gaagctagca ttgctgcagt gcaaacgccc tggt                                34


<210>  88
<211>  34
<212>  DNA
<213>  Gallus gallus

<400>  88
ggggctagcg gtggatgtgt ttgttttaca gagg                                34


<210>  89
<211>  34
<212>  DNA
<213>  Gallus gallus

<400>  89
gggactagtt gttcagatgg aacttcttat gttc                                34


<210>  90
<211>  34
<212>  DNA
<213>  Gallus gallus

<400>  90
ggggctagcg gtggatgtgt ttgttttaca gagg                                34


<210>  91
<211>  34
<212>  DNA
<213>  Gallus gallus

<400>  91

gaagctagca tgggatggaa gggcccgtct ggcc                                    34

<210>  92
<211>  34
<212>  DNA
<213>  Gallus gallus

<400>  92
gaagctagct ttatgctggg aacaggggga gttc                                    34

<210>  93
<211>  34
<212>  DNA
<213>  Gallus gallus

<400>  93
gaagctagca tgggatggaa gggcccgtct ggcc                                    34

<210>  94
<211>  34
<212>  DNA
<213>  Gallus gallus

<400>  94
gaagctagca ggactgtgct gtcctcatgc ccct                                    34

<210>  95
<211>  34
<212>  DNA
<213>  Gallus gallus

<400>  95
gaagctagca tgggatggaa gggcccgtct ggcc                                    34

<210>  96
<211>  34
<212>  DNA
<213>  Gallus gallus

<400>  96
gaagctagcc acgacagctg gggccacaca aaga                                    34

<210>  97
<211>  34
<212>  DNA
<213>  Gallus gallus

<400>  97
gaagctagca tgggatggaa gggcccgtct ggcc                                    34

<210>  98
<211>  34
<212>  DNA
<213>  Gallus gallus

<400>  98
gaagctagcg tcacaggttg taacaggctg acat                                    34

<210>  99
<211>  34

<212>  DNA
<213>  Gallus gallus

<400>  99
gaagctagca tgggatggaa gggcccgtct ggcc                                34


<210>  100
<211>  34
<212>  DNA
<213>  Gallus gallus

<400>  100
ggggctagct cacagaaaca ttgaaatggc tcct                                34


<210>  101
<211>  34
<212>  DNA
<213>  Gallus gallus

<400>  101
gaagctagca tgggatggaa gggcccgtct ggcc                                34


<210>  102
<211>  34
<212>  DNA
<213>  Gallus gallus

<400>  102
gaagctagct ttatgctggg aacaggggga gttc                                34


<210>  103
<211>  34
<212>  DNA
<213>  Gallus gallus

<400>  103
gaaactagta ttgctgcagt gcaaacgccc tggt                                34


<210>  104
<211>  40
<212>  DNA
<213>  Gallus gallus

<400>  104
aaatgatcac ccctctccct cccccccccc taacgttact                          40


<210>  105
<211>  40
<212>  DNA
<213>  Gallus gallus

<400>  105
gggtgatcag gatccgatcc agacatgata agatacattg                          40


<210>  106
<211>  41
<212>  DNA
<213>  Gallus gallus

<400>  106
gggggatcca gatctgtgac cggtgcaagt gatagaaaac t                        41

<210> 107
<211> 51
<212> DNA
<213> Gallus gallus

<400> 107
tacaaaaacc tcctgccact gcaaggagcg agctgatggt ttttactgtc t 51